# EUROPEAN PATENT APPLICATION

(11) **EP 4 317 408 A1**
(43) Date of publication of application: **07.02.2024**
(21) Application number: 22795587.9
(22) Date of filing: 13.04.2022
(51) Int. Cl.: C12M 3/02, C12M 1/34, C12N 1/00

(54) **CELL CULTURE SYSTEM AND METHOD FOR ADJUSTING VIABLE CELL CONCENTRATION**

(30) Priority: 28.04.2021 JP 2021076027
(71) Applicant: FUJIFILM Corporation, Tokyo 106-8620 (JP)
(72) Inventor: KAMEI, Shota, Ashigarakami-gun, Kanagawa 258-8577 (JP); MORIMOTO, Takashi, Ashigarakami-gun, Kanagawa 258-8577 (JP); SHIMODA, Tomoyuki, Ashigarakami-gun, Kanagawa 258-8577 (JP)
(74) Representative: Hoffmann Eitle
(86) International application number: PCT/JP2022/017731
(87) International publication number: WO 2022/230673

(57) **Abstract**

Provided are a cell culture apparatus and a living cell concentration adjustment method. The cell culture apparatus includes: a first container that holds a cell suspension; a second container that holds a diluent diluting the cell suspension; a living cell concentration measurement device that individually determines whether a plurality of target cells in the cell suspension are alive or dead to measure a living cell concentration of the target cells in the cell suspension; a control device that holds and controls the living cell concentration and amount data of the cell suspension; a mixing unit that mixes the cell suspension and the diluent; and a culture container in which the diluted cell suspension is seeded to culture cells. The control device determines an amount of the cell suspension to be seeded and an amount of the diluent required to adjust the cell suspension to be seeded to a predetermined living cell concentration, on the basis of the living cell concentration and the amount data of the cell suspension, feeds the amount of the cell suspension and the amount of the diluent to the mixing unit, and seeds the diluted cell suspension.

## Description

### BACKGROUND OF THE INVENTION

### 1. Field of the Invention

The present disclosure relates to a cell culture apparatus and a living cell concentration adjustment method.

### 2. Description of the Related Art

In a cell culture technique, it is necessary to appropriately adjust a living cell concentration of target cells in a cell suspension used for seeding. A technique related to the adjustment of the living cell concentration is disclosed in, for example, WO2016/013394A.

### SUMMARY OF THE INVENTION

In the related art, techniques related to the adjustment of the living cell concentration including the technique disclosed in WO2016/013394A have been examined. However, in the current situation, these techniques are not sufficient.

The present disclosure has been made in view of this situation, and an object of an embodiment of the present disclosure is to provide a cell culture apparatus that can rapidly adjust a living cell concentration of target cells.

An object of another embodiment of the present disclosure is to provide a living cell concentration adjustment method that can rapidly adjust a living cell concentration of target cells.

The present disclosure includes the following aspects.
<1> There is provided a cell culture apparatus comprising: a first container that holds a cell suspension; a second container that holds a diluent diluting the cell suspension; a living cell concentration measurement device that individually determines whether a plurality of target cells in the cell suspension are alive or dead to measure a living cell concentration of the target cells in the cell suspension; a control device that holds and controls the living cell concentration and amount data of the cell suspension; a mixing unit that mixes the cell suspension and the diluent; and a culture container in which the diluted cell suspension is seeded to culture cells. The control device determines an amount of the cell suspension to be seeded and an amount of the diluent required to adjust the cell suspension to be seeded to a predetermined living cell concentration, on the basis of the living cell concentration and the amount data of the cell suspension, feeds the amount of the cell suspension and the amount of the diluent to the mixing unit, and seeds the diluted cell suspension.
<2> In the cell culture apparatus according to <1>, the living cell concentration measurement device may be disposed in a flow path that is branched from a flow path between the first container and the mixing unit or in the flow path between the first container and the mixing unit.
<3> In the cell culture apparatus according to <2>, the living cell concentration may be measured for at least a portion of a liquid feeding time until the cell suspension reaches the mixing unit from the first container.
<4> In the cell culture apparatus according to any one of <1> to <3>, the mixing unit may also serve as the culture container.
<5> In the cell culture apparatus according to any one of <1> to <4>, the mixing unit may also serve as the culture container which is a suspension culture apparatus.
<6> In the cell culture apparatus according to any one of <1> to <3>, the mixing unit may also serve as the second container.
<7> In the cell culture apparatus according to any one of <1> to <6>, the culture container may be a multi-layer culture container.
<8> In the cell culture apparatus according to any one of <1> to <7>, the living cell concentration measurement device may include: a light source that irradiates the target cell with light; an imaging device that images the target cell; a unit that changes a focal plane; an image acquisition unit that acquires images of the target cell captured in a plurality of focal planes including an in-focus plane of the target cell in a direction opposite to a side on which the target cell is irradiated with the light; an image piece acquisition unit that acquires an image piece including a central portion and an outer peripheral portion of the target cell from each of the images; a connected-image-for-analysis creation unit that connects the image pieces in an order of an imaging direction of the focal plane to create a connected image for analysis; a feature amount extraction unit that extracts a feature amount from the connected image for analysis; a life-and-death determination unit that determines whether the plurality of target cells in the cell suspension are alive or dead on the basis of the feature amount of the connected image for analysis and a predetermined range of the feature amount; and a living cell concentration determination unit that determines the living cell concentration of the target cells in the cell suspension on the basis of a result of determining whether the target cells are alive or dead.
<9> In the cell culture apparatus according to <8>, the unit changing the focal plane may be a stage moving mechanism that moves a stage, on which a holding container holding the target cell is placed, to change a distance between the target cell and the imaging device.
<10> In the cell culture apparatus according to <8>, the unit changing the focal plane may be an imaging device moving mechanism that moves the imaging device to change a distance between the target cell and the imaging device.
<11> In the cell culture apparatus according to <8>, the imaging device may include a liquid lens as the unit changing the focal plane.
<12> In the cell culture apparatus according to any one of <1> to <7>, the living cell concentration measurement device may include: a digital holographic microscope that images the target cell; a phase image acquisition unit that acquires a phase image of the target cell; a life-and-death determination unit that determines whether the plurality of target cells in the cell suspension are alive or dead on the basis of a phase amount distribution of the phase image and a predetermined phase amount distribution; and a living cell concentration determination unit that determines the living cell concentration of the target cells in the cell suspension on the basis of a result of determining whether the target cells are alive or dead.
<13> In the cell culture apparatus according to any one of <1> to <12>, the living cell concentration measurement device may include a cell survival rate determination unit that determines a cell survival rate of the target cells in the cell suspension on the basis of a result of determining whether the target cells are alive or dead.
<14> There is provided a living cell concentration adjustment method comprising: acquiring images of a target cell captured in a plurality of focal planes including an in-focus plane of the target cell in a direction opposite to a side on which the target cell is irradiated with light; individually determining whether a plurality of the target cells in a cell suspension are alive or dead to measure a living cell concentration of the target cells in the cell suspension; determining an amount of the cell suspension to be seeded and an amount of a diluent required to adjust the cell suspension to be seeded to a predetermined living cell concentration, on the basis of the living cell concentration and amount data of the cell suspension; and mixing the amount of the cell suspension and the amount of the diluent.
<15> In the living cell concentration adjustment method according to <14>, the measuring of the living cell concentration may include: acquiring images of the target cell captured in a plurality of focal planes including an in-focus plane of the target cell in a direction opposite to a side on which the target cell is irradiated with the light; acquiring an image piece including a central portion and an outer peripheral portion of the target cell from each of the images; connecting the image pieces in an order of an imaging direction of the focal plane to create a connected image for analysis; extracting a feature amount from the connected image for analysis; determining whether the plurality of target cells in the cell suspension are alive or dead on the basis of the feature amount of the connected image for analysis and a predetermined range of the feature amount; and determining the living cell concentration of the target cells in the cell suspension on the basis of a result of determining whether the target cells are alive or dead.
<16> In the living cell concentration adjustment method according to <14>, the measuring of the living cell concentration may include: imaging the target cell with a digital holographic microscope to acquire a phase image of the target cell; determining whether the plurality of target cells in the cell suspension are alive or dead on the basis of a phase amount distribution of the phase image and a predetermined phase amount distribution; and determining the living cell concentration of the target cells in the cell suspension on the basis of a result of determining whether the target cells are alive or dead.
<17> The living cell concentration adjustment method according to any one of <14> to <16> may further comprise: determining a cell survival rate of the target cells in the cell suspension on the basis of a result of determining whether the target cells are alive or dead.
<18> In the cell culture apparatus according to any one of <1> to <13>, the target cell may be a human synovium-derived mesenchymal stem cell.
<19> There is provided a method for producing a therapeutic agent for arthropathy using the living cell concentration adjustment method according to any one of <14> to <17>, in which the target cell is a human synovium-derived mesenchymal stem cell.
<20> There is provided a therapeutic agent for arthropathy that is produced by the method for producing a therapeutic agent for arthropathy according to <19>.

According to one embodiment of the present disclosure, there is provided a cell culture apparatus that can rapidly adjust a living cell concentration of target cells.

According to another embodiment of the present disclosure, there is provided a living cell concentration adjustment method that can rapidly adjust a living cell concentration of target cells.

### BRIEF DESCRIPTION OF THE DRAWINGS

Fig. 1 is a schematic diagram illustrating an example of a cell culture apparatus.
Fig. 2 is a block diagram illustrating an example of a control device that constitutes the cell culture apparatus.
Fig. 3 is a schematic diagram illustrating an example of the cell culture apparatus.
Fig. 4 is a schematic diagram illustrating an example of the cell culture apparatus.
Fig. 5 is a schematic diagram illustrating an example of the cell culture apparatus.
Fig. 6 is a schematic diagram illustrating an example of the cell culture apparatus.
Fig. 7 is a schematic diagram illustrating an example of the cell culture apparatus.
Fig. 8 is a schematic diagram illustrating an example of the cell culture apparatus.
Fig. 9 is a schematic diagram illustrating an example of the cell culture apparatus.
Fig. 10 is a schematic diagram illustrating an example of a living cell concentration measurement device.
Fig. 11 is a schematic diagram illustrating an example of focal planes.
Fig. 12 is a schematic diagram illustrating an example of an image obtained by imaging a living cell while changing the focal plane.
Fig. 13 is a schematic diagram illustrating an example of an image obtained by imaging a dead cell while changing the focal plane.
Fig. 14 is a schematic diagram illustrating an example of a connected image obtained from the living cell.
Fig. 15 is a schematic diagram illustrating an example of a connected image obtained from the dead cell.
Fig. 16 is a schematic diagram illustrating an example of a process of a machine learning device in a learning phase and an operation phase.
Fig. 17 is a flowchart illustrating an example of a life-and-death determination flow of a life-and-death determination step.
Fig. 18 is a block diagram illustrating an example of a control unit that constitutes a cell life-and-death determination device.
Fig. 19 is a block diagram illustrating an example of a process of the cell life-and-death determination device.

### DESCRIPTION OF THE PREFERRED EMBODIMENTS

Hereinafter, a cell culture apparatus and a living cell concentration adjustment method according to the present disclosure will be described in detail.

A numerical range described using "to" in the present disclosure means a range that includes numerical values written before and after "to" as a minimum value and a maximum value, respectively.

In the numerical range described stepwise in the present disclosure, an upper limit value or a lower limit value described in a certain numerical range may be replaced with an upper limit value or a lower limit value of another numerical range described stepwise. Further, in the numerical range described in the present disclosure, an upper limit value or a lower limit value disclosed in a certain range may be replaced with values described in examples.

In the present disclosure, a combination of two or more preferred aspects is a more preferred aspect.

In the present disclosure, in a case in which a plurality of types of substances corresponding to each component are present, the amount of each component means the total amount of the plurality of types of substances, unless otherwise specified.

In the present disclosure, the term "step" includes not only an independent step but also a step of which an intended purpose is achieved even in a case in which the step is not clearly distinguishable from other steps.

The drawings referred to in the following description are exemplarily and schematically illustrated, and the present disclosure is not limited to the drawings. The same reference numerals denote the same components. Further, reference numerals in the drawings may be omitted.

### <Cell Culture Apparatus>

A cell culture apparatus according to the present disclosure includes: a first container that holds a cell suspension; a second container that holds a diluent diluting the cell suspension; a living cell concentration measurement device that individually determines whether a plurality of target cells in the cell suspension are alive or dead to measure a living cell concentration of the target cells in the cell suspension; a control device that holds and controls the living cell concentration and amount data of the cell suspension; a mixing unit that mixes the cell suspension and the diluent; and a culture container in which the diluted cell suspension is seeded to culture cells. The control device determines an amount of the cell suspension to be seeded and an amount of the diluent required to adjust the cell suspension to be seeded to a predetermined living cell concentration, on the basis of the living cell concentration and the amount data of the cell suspension, feeds the amount of the cell suspension and the amount of the diluent to the mixing unit, and seeds the diluted cell suspension.

The technique disclosed in WO2016/013394A is given as an example of the technique related to the adjustment of the living cell concentration. In the technique disclosed in WO2016/013394A, in a case in which the living cell concentration is measured, a calibration curve prepared in advance is used. Therefore, it is necessary to prepare a large number of calibration curves in consideration of, for example, the type of target cell and the mixture of other cell types in a cell suspension. In addition, in the technique disclosed in WO2016/013394A, in a case in which the cell suspension and a diluent are mixed, it is necessary to mix the cell suspension and the diluent using a circulation path. Therefore, in the technique disclosed in WO2016/013394A, it takes a lot of time to adjust the living cell concentration.

On the other hand, in the cell culture apparatus according to the present disclosure, it is individually determined whether a plurality of target cells in the cell suspension are alive or dead. Therefore, it is possible to measure the living cell concentration of the target cells (hereinafter, sometimes simply referred to as a "living cell concentration") and to rapidly adjust the living cell concentration.

In a case in which cells derived from biological tissues are used, for example, in a case in which cells derived from human tissues are used, for example, in the production of a cell preparation from the cells derived from the human tissues, there is a limit to the amount of cells that can be collected, and rapid cell preparation is required not to deteriorate the performance of the preparation. Specifically, the cell culture apparatus according to the present disclosure can also be used in the production of a cell preparation as a therapeutic agent for arthropathy from human synovium-derived mesenchymal stem cells.

The "target cell" means a specific type of cell to be determined for life or death and may be, for example, a cell used for cell culture or the like.

The "predetermined living cell concentration" may be, for example, a target living cell concentration determined for a cell suspension of the target cells used for seeding.

The type of target cell is not particularly limited, and it is possible to determine whether various living cells are alive or dead. Specifically, the target cell may be a mesenchymal stem cell collected or isolated from a biological tissue, and an example of the target cell is the human synovium-derived mesenchymal stem cell. Meanwhile, the synovium-derived mesenchymal stem cell is also referred to as a synovium-derived stem cell.

Hereinafter, each configuration will be described in detail.

### [First Container]

The first container holds the cell suspension. The amount of cell suspension determined on the basis of the living cell concentration and the amount data of the cell suspension is fed to the mixing unit.

For example, the material and shape of the first container are not particularly limited and may be appropriately selected in consideration of the configuration of the cell culture apparatus. The type of target cell is not particularly limited. The living cell concentrations of various target cells can be measured, and then the target cells can be diluted and seeded. From the viewpoint of facilitating the feeding of the cell suspension at a uniform concentration, the first container may comprise a mixing mechanism, such as a stirrer, and a shaking mechanism that can shake the first container.

### [Second Container]

The second container holds the diluent that dilutes the cell suspension. The amount of diluent determined on the basis of the living cell concentration and the amount data of the cell suspension is fed to the mixing unit.

For example, the material and shape of the second container are not particularly limited and may be appropriately selected in consideration of the configuration of the cell culture apparatus. The type of diluent is not particularly limited and may be appropriately selected in consideration of the type of target cell. In addition, the mixing unit may also serve as the second container, which will be described below.

### [Living Cell Concentration Measurement Device]

The living cell concentration measurement device individually determines whether the plurality of target cells in the cell suspension are alive or dead to measure the living cell concentration of the target cells in the cell suspension.

The living cell concentration measurement device may comprise a cell survival rate determination unit that determines a cell survival rate of the target cells in the cell suspension on the basis of the result of determining whether the plurality of target cells in the cell suspension are alive or dead. The cell survival rate determination unit of the living cell concentration measurement device corresponds to a cell survival rate determination step of a living cell concentration adjustment method, which will be described in detail below.

The living cell concentration measurement device may be disposed at any position of the cell culture apparatus. From the viewpoint of measuring the living cell concentration without exposing the cell suspension to an external environment, it is preferable that the living cell concentration measurement device is disposed in a flow path that is branched from a flow path between the first container and the mixing unit or in the flow path between the first container and the mixing unit (hereinafter, sometimes referred to as "the living cell concentration measurement device is in-line"). Therefore, the cell suspension can be kept in a cleaner state.

In a case in which the living cell concentration measurement device is in-line, the living cell concentration may be measured for at least a portion of a liquid feeding time until the cell suspension reaches the mixing unit from the first container. The living cell concentration can be more rapidly adjusted by feeding the cell dispersion to the mixing unit while measuring the living cell concentration.

A method for measuring the living cell concentration is not particularly limited.

A living cell concentration measurement device (hereinafter, sometimes referred to as a "living cell concentration measurement device according to Aspect 1") that analyzes a connected image obtained from a plurality of images of the target cell to determine whether the target cell is alive or dead and measures the living cell concentration is given as an example, which will be described in detail below.

In addition, a living cell concentration measurement device (hereinafter, sometimes referred to as a "living cell concentration measurement device according to Aspect 2") that analyzes a phase image of the target cell captured by a digital holographic microscope to determine whether the target cell is alive or dead and measures the living cell concentration is given as an example.

Hereinafter, the living cell concentration measurement devices according to Aspects 1 and 2 will be specifically described.

### (Living Cell Concentration Measurement Device According to Aspect 1)

The living cell concentration measurement device according to Aspect 1 comprises: a light source that irradiates the target cell with light; an imaging device that images the target cell; a unit that changes a focal plane; an image acquisition unit that acquires images of the target cell captured in a plurality of focal planes including an in-focus plane of the target cell in a direction opposite to a side on which the target cell is irradiated with the light; an image piece acquisition unit that acquires an image piece including a central portion and an outer peripheral portion of the target cell from each of the images; a connected-image-for-analysis creation unit that connects the image pieces in an order of an imaging direction of the focal plane to create a connected image for analysis; a feature amount extraction unit that extracts a feature amount from the connected image for analysis; a life-and-death determination unit that determines whether the plurality of target cells in the cell suspension are alive or dead on the basis of the feature amount of the connected image for analysis, a feature amount of a known connected image for reference of the target cell, and a result of determining whether or not the target cell is the living cell; and a living cell concentration determination unit that determines the living cell concentration of the target cells in the cell suspension on the basis of a result of determining whether the target cells are alive or dead.

Since the living cell is covered with a cell membrane, the living cell has a property of having a spherical shape in the cell suspension (in addition, the spherical shape does not mean a perfect spherical shape). In addition, the living cell has a translucent property. Therefore, the living cell has a property of a spherical lens, and this is called a "lens effect".

Therefore, in a case in which the living cell is irradiated with light, a focusing point (hereinafter, sometimes referred to as a "focusing point of the lens effect") is formed in a direction opposite to the side, on which the living cell is irradiated with light, by the lens effect. In a case in which the living cell is imaged in the direction opposite to the side on which the living cell is irradiated with light, it is possible to acquire an image including the focusing point of the lens effect in the in-focus plane of the living cell.

Therefore, a connected image created from the images of the living cell acquired from a plurality of focal planes including the in-focus plane includes information unique to the living cell.

On the other hand, a dead cell does not have a spherical shape because a cell membrane is broken. In addition, a liquid medium of the cell suspension flows into the dead cell. Therefore, the dead cell does not exhibit the lens effect.

Therefore, even in a case in which the dead cell is irradiated with light, the focusing point of the lens effect is not formed. As a result, even in a case in which the dead cell is imaged in the in-focus plane of the dead cell, it is not possible to acquire an image including the focusing point of the lens effect.

As described above, since the connected image created from the living cell is different from the connected image created from the dead cell, it has a feature amount unique to the living cell. Therefore, it is possible to determine whether the target cell is alive or dead on the basis of the feature amount of the connected image for analysis of the target cell whose life or death is unknown and a predetermined range of the feature amount.

In addition, since the cell culture apparatus according to Aspect 1 uses the optical properties of cells, it is possible to more easily adjust the living cell concentration without using a staining reagent.

In some cases, the connected image for analysis and the connected image for reference are simply referred to as a "connected image". In addition, in some cases, the "target cell" is simply referred to as a "cell".

The life-and-death determination unit may be configured by a machine learning device.

The image acquisition unit, the image piece acquisition unit, the connected-image-for-analysis creation unit, the feature amount extraction unit, the life-and-death determination unit, and the living cell concentration determination unit in the living cell concentration measurement device according to Aspect 1 correspond to an image acquisition step, an image piece acquisition step, a connected-image-for-analysis creation step, a feature amount extraction step, a life-and-death determination step, and a living cell concentration determination step in a living cell concentration measurement step according to Aspect 1 which will be described in detail below, respectively.

The imaging device consists of a combination of an imaging lens and an area sensor. The imaging lens may be, for example, a telecentric lens or a microscope objective lens.

The light source is not particularly limited, and an example of the light source is a light emitting diode (LED).

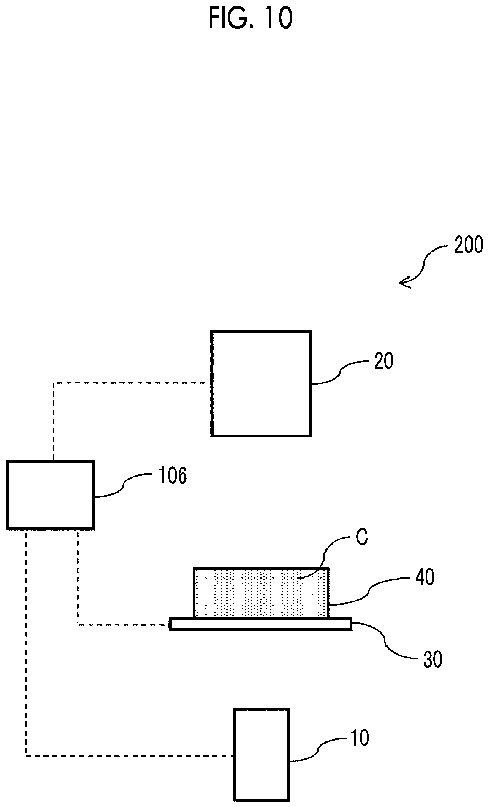

The unit that changes the focal plane is not particularly limited and may be, for example, a stage moving mechanism that moves a stage, on which a holding container holding the cell is placed, to change a distance between the cell and the imaging device. As illustrated in Fig. 10, for example, a living cell concentration measurement device 200 may comprise a light source 10 that irradiates a target cell C with light, an imaging device 20 that images the target cell C, a holding container 40 that holds the target cell (cell suspension) C, a stage 30 (stage moving mechanism) that moves the holding container 40 to change the distance between the target cell C and the imaging device 20, and a control unit 106.

The unit that changes the focal plane may be an imaging device moving mechanism that moves the imaging device to change the distance between the cell and the imaging device.

In addition, the imaging device may comprise a liquid lens as the unit that changes the focal plane.

The living cell concentration measurement device may comprise an input device for inputting data and a display device for displaying, for example, a measurement result of the living cell concentration.

The living cell concentration measurement device may be configured such that data can be input through an input device comprised in the cell culture apparatus. In addition, the living cell concentration measurement device may be configured such that, for example, the measurement result of the living cell concentration can be displayed on a display device comprised in the cell culture apparatus.

Hereinafter, a living cell concentration measurement device will be described in more detail using the living cell concentration measurement device 200 illustrated in Fig. 10 as an example.

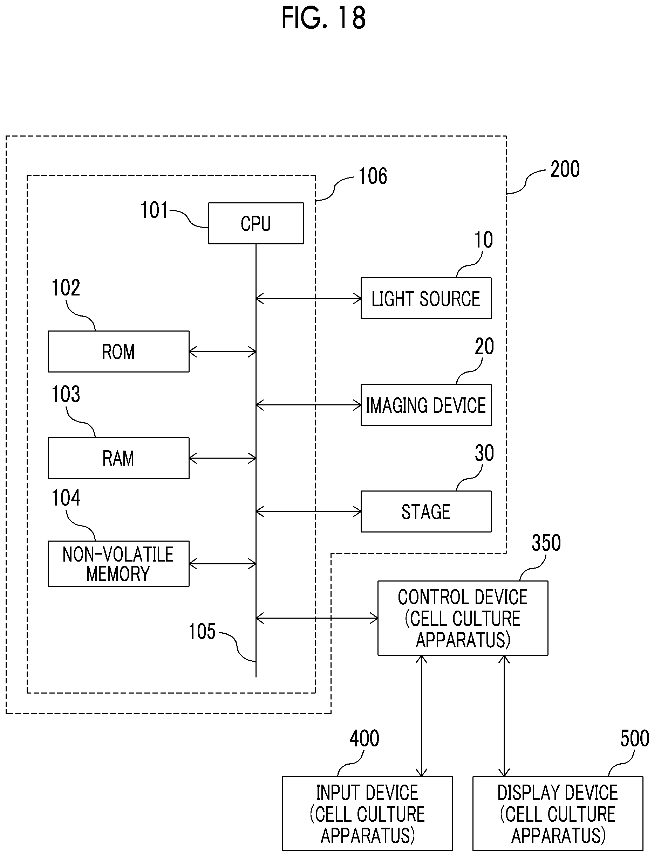

Fig. 18 is a block diagram illustrating an example of the control unit 106 constituting the living cell concentration measurement device 200 illustrated in Fig. 10. A central processing unit (CPU) 101 comprised in the control unit 106 is a processor that controls the overall operation of the living cell concentration measurement device 200. The CPU 101 reads a system program stored in a read only memory (ROM) 102 through a bus 105 and controls the entire living cell concentration measurement device 200 according to the system program. A random access memory (RAM) 103 temporarily stores, for example, temporary calculation data, data to be displayed on a display device 500 of a control device 350 of the cell culture apparatus, and various types of data input through an input device 400 of the control device 350 of the cell culture apparatus.

A non-volatile memory 104 stores, for example, data acquired from the light source 10, data acquired from the imaging device 20, data acquired from the stage 30, and data inputted from the input device 50, using a static random access memory (SRAM), a solid state drive (SSD), or the like which is backed up by a battery (not illustrated). For example, the data and the programs stored in the non-volatile memory 104 may be developed in the RAM 103 during use. In addition, for example, various algorithms required to analyze image data acquired from the imaging device 20 and system programs for performing other required processes are written in the ROM 102 in advance.

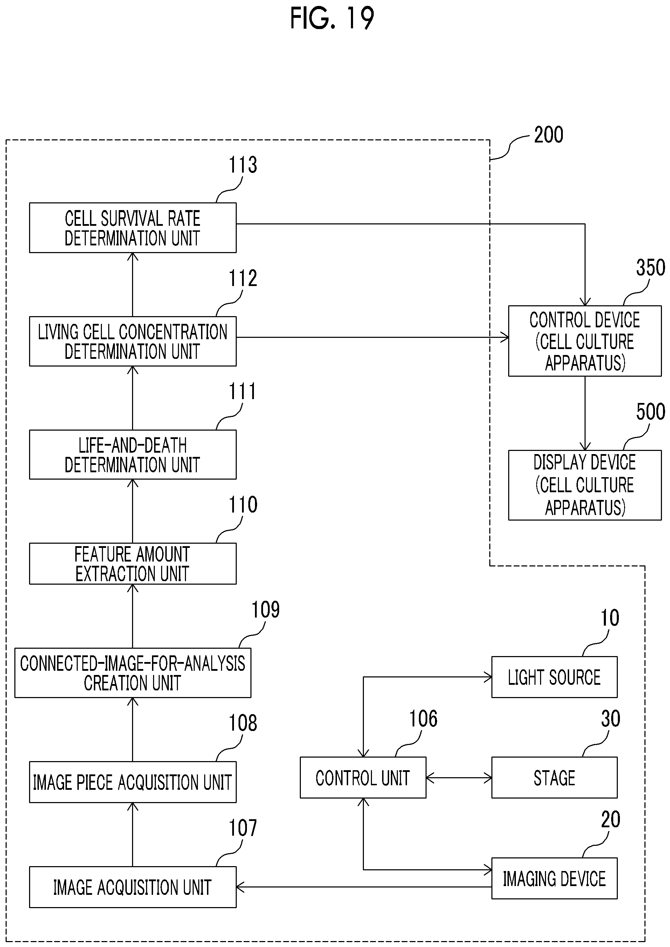

Fig. 19 is a block diagram illustrating an example of a process of the living cell concentration measurement device illustrated in Fig. 10. The CPU 101 comprised in the control unit 106 illustrated in Fig. 10 executes the system program to control the operation of each unit of the living cell concentration measurement device 200, thereby implementing each functional block illustrated in Fig. 19.

The control unit 106 controls the light source 10, the imaging device 20, and the stage 30 on the basis of an imaging program stored in the non-volatile memory 104 such that the cell C is imaged. The control unit 106 turns on the light source 10 to irradiate the cell C with light and drives the stage 30 such that the holding container 40 holding the cell (cell suspension) C is moved to change the focal plane. After moving the stage 30 to a predetermined focal plane, the control unit 106 issues a command to perform an imaging operation to the imaging device 20. The control unit 106 images the cell in a plurality of focal planes including the in-focus plane of the cell in the direction opposite to the side on which the cell is irradiated with the light according to the imaging program.

An image acquisition unit 107 acquires the image of the target cell captured by the imaging device 20. For the image of the target cell acquired by the image acquisition unit 107, a plurality of images obtained by imaging one target cell may be collectively managed as one set of image data groups.

An image piece acquisition unit 108 performs image processing on the image of the target cell acquired by the image acquisition unit 107 and acquires an image piece including a central portion and an outer peripheral portion of the target cell from the image of the target cell. For the image piece acquired by the image piece acquisition unit 108, a plurality of image pieces obtained by imaging one target cell may be collectively managed as one set of image piece data groups.

A connected-image-for-analysis creation unit 109 performs image processing on the image pieces obtained by the image piece acquisition unit 108 and connects the image pieces in the order of the imaging direction of the focal plane to create a connected image for analysis.

A feature amount extraction unit 110 performs image processing on the connected image for analysis obtained by the connected-image-for-analysis creation unit 109 to extract a feature amount from the connected image for analysis.

A life-and-death determination unit 111 determines whether the cell is alive or dead on the basis of the feature amount of the connected image for analysis extracted by the feature amount extraction unit 110 and a predetermined range of the feature amount. In another embodiment, the life-and-death determination unit 111 determines whether the cell is alive or dead on the basis of the feature amount of the connected image for analysis extracted by the feature amount extraction unit 110, the feature amount of a known connected image for reference of the target cell, and the result of determining whether or not the target cell is the living cell.

A living cell concentration determination unit 112 determines the living cell concentration of the target cell on the basis of the result of determining whether the target cell is alive or dead, that is, the number of connected images for analysis in which the target cell has been determined to be the living cell. The living cell concentration determination unit 112 outputs the living cell concentration of the target cell to the display device 500 through the control device 350 of the cell culture apparatus.

A cell survival rate determination unit 113 determines a cell survival rate on the basis of the result of determining whether the target cell is alive or dead, that is, the number of connected images for analysis in which the target cell has been determined to be the living cell and the total number of connected images for analysis. The cell survival rate determination unit 113 outputs the cell survival rate to the display device 500 through the control device 350 of the cell culture apparatus.

### (Living Cell Concentration Measurement Device According to Aspect 2)

The living cell concentration measurement device according to Aspect 2 comprises: a digital holographic microscope that images the target cell; a phase image acquisition unit that acquires a phase image of the target cell; a life-and-death determination unit that determines whether the plurality of target cells in the cell suspension are alive or dead on the basis of a phase amount distribution of the phase image and a predetermined phase amount distribution; and a living cell concentration determination unit that determines the living cell concentration of the target cells in the cell suspension on the basis of a result of determining whether the target cells are alive or dead.

The living cell has a difference in refractive index between the inside of the cell and the periphery of the cell, which is a source of the lens effect, and also has a refractive index distribution. On the other hand, for the dead cell, since the cell membrane is broken, the liquid medium of the cell suspension flows into the cell. Therefore, the difference in refractive index between the inside of the cell and the periphery of the cell is small, and the refractive index distribution is also small. Since an internal refractive index can be detected as the phase amount, the phase image of the target cell obtained by the digital holographic microscope has a phase amount distribution unique to the living cell.

Therefore, it is possible to determine whether the target cell is alive or dead on the basis of the phase amount distribution of the phase image of the target cell whose life or death is unknown and a predetermined range of the phase amount distribution.

In addition, since the cell culture apparatus according to Aspect 2 uses the optical properties of cells, it is possible to more easily adjust the living cell concentration without using a staining reagent.

The control unit of the living cell concentration measurement device according to Aspect 2 may be appropriately designed in the same manner as that in Aspect 1.

The phase image acquisition unit, the life-and-death determination unit, and the living cell concentration determination unit in the living cell concentration measurement device according to Aspect 2 correspond to a phase image acquisition step, a life-and-death determination step, and a living cell concentration determination step in a living cell concentration measurement step according to Aspect 2 which will be described in detail below, respectively.

The target cell is imaged by the digital holographic microscope. The phase image acquisition unit acquires the captured phase image of the target cell.

The living cell concentration measurement devices according to Aspects 1 and 2 have been described above. However, other details are as described below in a living cell concentration adjustment method.

### [Control Device]

The control device holds and controls the living cell concentration and the amount data of the cell suspension.

The control device determines the amount of cell suspension to be seeded and the amount of diluent required to adjust the cell suspension to be seeded to a predetermined living cell concentration on the basis of the living cell concentration and the amount data of the cell suspension. Then, the control device feeds the amount of cell suspension and the amount of diluent to the mixing unit to seed the diluted cell suspension.

### [Mixing Unit]

The mixing unit mixes the cell suspension and the diluent.

In a certain embodiment, the mixing unit may be a container that mixes the amount of cell suspension and the amount of diluent determined on the basis of the living cell concentration and the amount data of the cell suspension. The container as the mixing unit may comprise a mixing mechanism, such as a stirrer, and a shaking mechanism that can shake the mixing unit. The diluted cell dispersion is fed to the culture container.

Examples of the aspect of the shaking include rotation, shaking in a vertical direction, and shaking in a horizontal direction.

The shaking mechanism is not particularly limited, and examples of the shaking mechanism include a cam mechanism, a link mechanism, and a linear motion mechanism. More specifically, examples of the shaking mechanism include an articulated robot, a uniaxial robot, a combination of linear motion cylinders, and a shaker.

The aspect of the shaking and the shaking mechanism are the same in the description of other configurations according to the present disclosure.

In another embodiment, the mixing unit may also serve as the culture container. That is, the culture container may have a function of mixing the cell suspension and the diluent.

In a case in which the mixing unit also serves as the culture container, the "feeding of the cell culture and the diluent to the mixing unit" means the feeding of the cell culture and the diluent to the culture container that also serves as the mixing unit.

In a case in which the mixing unit also serves as the culture container, the mixing unit may be, for example, a shaking mechanism that can shake the culture container. This makes it possible to seed the diluted cell suspension while mixing the cell suspension and the diluent. Examples of the aspect of the shaking include rotation, shaking in the vertical direction, and shaking in the horizontal direction.

The mixing unit may also serve as the culture container which is a suspension culture apparatus. In a case in which the mixing unit also serves as the culture container which is the suspension culture apparatus, the mixing unit may be, for example, a circulation mechanism that can circulate the diluted cell suspension. The suspension culture apparatus is a culture apparatus that can culture cells while circulating and suspending the cells. This makes it possible to seed the diluted cell suspension while mixing the cell suspension and the diluent. An example of the circulation mechanism is a stirring mechanism.

In another embodiment, the mixing unit may also serve as the second container. That is, the second container may have a function of mixing the cell suspension and the diluent.

In a case in which the mixing unit also serves as the culture container, the "feeding of the cell culture and the diluent to the mixing unit" means the feeding of the cell culture to the culture container that also serves as the mixing unit and holds the diluent.

For example, the material and shape of the mixing unit are not particularly limited and may be appropriately selected in consideration of the configuration of the cell culture apparatus.

### [Culture Container]

The culture container is for seeding the diluted cell suspension to culture cells.

In order to facilitate seeding, the culture container may comprise a shaking mechanism that can shake the culture container.

The culture container may be a multi-layer culture container. The multi-layer culture container is a culture container comprising a plurality of layers that can seed and culture cells.

In addition, as described above, the culture container may be the suspension culture apparatus that also serves as the mixing unit.

The cell culture apparatus comprises a flow path that connects the first container, the second container, the living cell concentration measurement device, the control device, a mixing unit 330, and the culture container. In the present disclosure, the flow path is not denoted by a reference numeral in order to simplify the drawings.

The cell culture apparatus may comprise, for example, a valve, a pump, and a flow meter in the flow path. For example, the adjustment of the degree of opening and closing of the valve and the adjustment of the output of the pump make it easier to control a flow rate in the flow path. In addition, the use of the flow meter makes it possible to easily understand the flow rate at a desired position in the flow path.

The valve is not particularly limited and may be, for example, a pinch valve. Further, the pump is not particularly limited and may be, for example, a peristaltic pump.

The cell culture apparatus may comprise a waste liquid container for discarding the cell suspension that has been used to measure the living cell concentration. The waste liquid container is not particularly limited, and examples of the waste liquid container include a bag, a bottle, and a syringe.

The cell culture apparatus may comprise an input device for inputting data and a display device for displaying, for example, the living cell concentration obtained from the living cell concentration measurement device and the living cell concentration of the diluted cell suspension.

### (Embodiment 1)

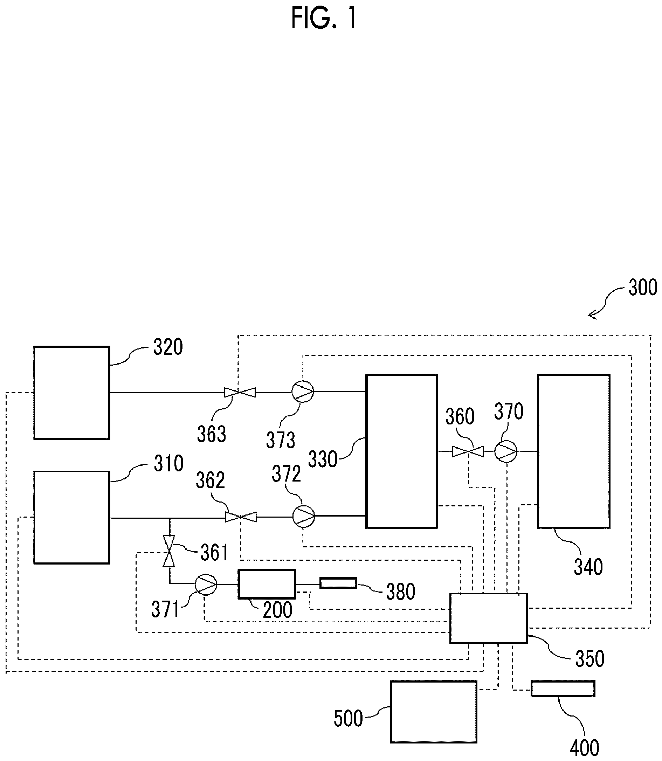

Hereinafter, the cell culture apparatus according to the present disclosure will be described in more detail using a cell culture apparatus 300 illustrated in Fig. 1 as an example.

The cell culture apparatus 300 illustrated in Fig. 1 comprises a first container 310, a second container 320, a living cell concentration measurement device 200, a control device 350, a mixing unit 330, and a culture container 340. Further, the cell culture apparatus 300 comprises an input device 400 and a display device 500.

The first container 310 and the mixing unit 330 are connected by a flow path through a valve 362 and a pump 372. In addition, the second container 320 and the mixing unit 330 are connected by a flow path through a valve 363 and a pump 373. Further, the mixing unit 330 and the culture container 340 are connected by a flow path through a valve 360 and a pump 370.

The first container 310 and the living cell concentration measurement device 200 are connected by a flow path through a valve 361 and a pump 371. In addition, the living cell concentration measurement device 200 is disposed in a flow path that is branched from the flow path between the first container 310 and the mixing unit 330. Furthermore, a waste liquid container 380 is connected to the living cell concentration measurement device 200.

The cell culture apparatus may comprise an input device for inputting data and a display device for displaying, for example, the measurement result of the living cell concentration. For example, a keyboard can be used as the input device. For example, a monitor can be used as the display device.

The control device 350 is connected to the first container 310, the second container 320, the mixing unit 330, the culture container 340, the valves 360 to 363, the pumps 371 to 373, the living cell concentration measurement device 200, the input device 400, and the display device 500 such that it can communicate therewith.

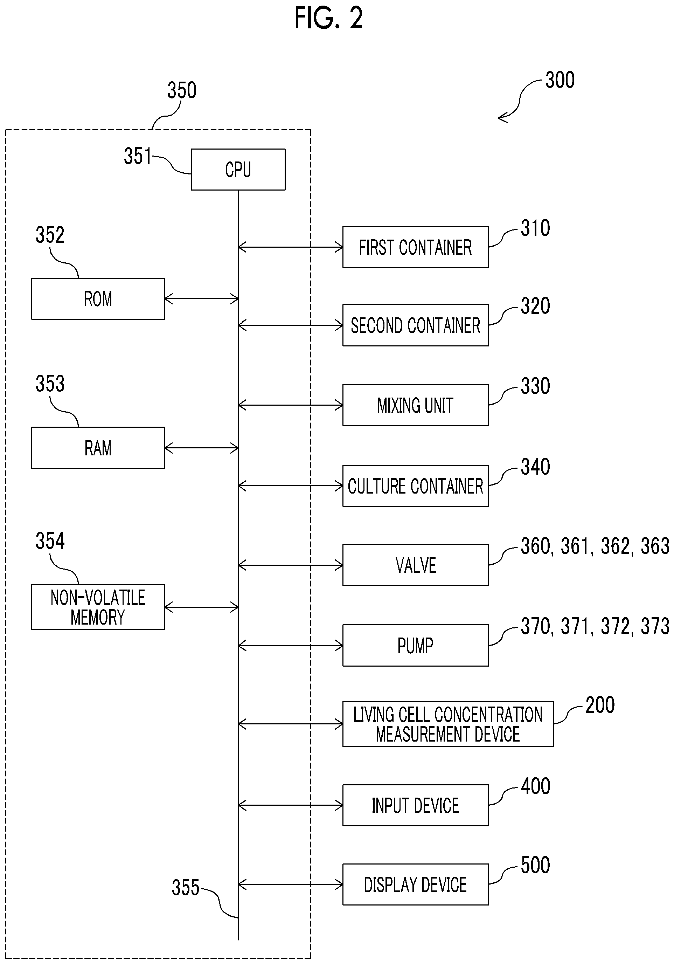

Fig. 2 is a block diagram illustrating an example of the control device 350 constituting the cell culture apparatus 300 illustrated in Fig. 1. A central processing unit (CPU) 351 included in the control device 350 is a processor that controls the overall operation of the cell culture apparatus 300. The CPU 351 reads a system program stored in a read only memory (ROM) 352 through a bus 355 and controls the entire cell culture apparatus 300 according to the system program. A random access memory (RAM) 353 temporarily stores, for example, temporary calculation data, data to be displayed on the display device 500, and various types of data input through the input device 400.

For example, a non-volatile memory 354 stores data, such as the amount data of the cell suspension acquired from the first container 310, data, such as the amount data of the diluent acquired from the second container 320, and data, such as the living cell concentration acquired from the living cell concentration measurement device 200, using a static random access memory (SRAM), a solid state drive (SSD), or the like which is backed up by a battery (not illustrated). In addition, the non-volatile memory 354 stores, for example, data acquired from the mixing unit 330, data acquired from the culture container 340, data acquired from the valves 360 to 363, data acquired from the pumps 371 to 373, and data input from the input device 400. For example, the data and programs stored in the non-volatile memory 354 may be developed in the RAM 353 during use. Further, for example, various algorithms, which are required to determine the amount of cell suspension to be seeded and the amount of diluent required to adjust the cell suspension to be seeded to a predetermined living cell concentration on the basis of the living cell concentration and the amount data of the cell suspension, and system programs for performing other required processes are written in the ROM 352 in advance.

The following Cell Culture Examples 1 and 2 will be described using the cell culture apparatus 300 as an example.

### -Cell Culture Example 1-

The control device 350 opens the valve 361 to drive the pump 371 such that the cell suspension is fed from the first container 310 to the living cell concentration measurement device 200 and then closes the valve 361 to stop the pump 371.

The living cell concentration of the cell suspension is measured by the living cell concentration measurement device 200, and the cell suspension that has been used for the measurement is discharged to the waste liquid container 380.

The control device 350 acquires the living cell concentration of the cell suspension from the living cell concentration measurement device 200 and acquires the amount data of the cell suspension from the first container 310.

The control device 350 determines the amount of cell suspension to be seeded and the amount of diluent required to adjust the cell suspension to be seeded to a predetermined living cell concentration on the basis of the living cell concentration and the amount data of the cell suspension.

The control device 350 opens the valve 362 to drive the pump 372 such that the determined amount of cell suspension is fed from the first container 310 to the mixing unit 330 and then closes the valve 362 to stop the pump 372.

In addition, the control device 350 opens the valve 363 to drive the pump 373 such that the determined amount of diluent is fed from the second container 320 to the mixing unit 330 and then closes the valve 363 to stop the pump 373.

The control device 350 drives a mixing mechanism (not illustrated) comprised in the mixing unit 330 to mix the cell suspension and the diluent such that the cell suspension is diluted.

The control device 350 opens the valve 360 to drive the pump 370 such that the diluted cell suspension is fed from the mixing unit 330 to the culture container 340 and is seeded in the culture container 340.

### -Cell Culture Example 2-

The control device 350 opens the valve 362 to drive the pump 372 at the same time as opening the valve 361 to drive the pump 371. Then, the control device 350 starts the feeding of the cell suspension from the first container 310 to the mixing unit 330 while feeding the cell suspension from the first container 310 to the living cell concentration measurement device 200. After feeding the cell suspension to the living cell concentration measurement device 200, the control device 350 closes the valve 361 to stop the pump 371.

The living cell concentration of the cell suspension is measured by the living cell concentration measurement device 200 for at least a portion of the liquid feeding time until the cell suspension reaches the mixing unit 330 from the first container 310. The cell suspension that has been used for the measurement is discharged to the waste liquid container 380.

The control device 350 acquires the living cell concentration of the cell suspension from the living cell concentration measurement device 200 and acquires the amount data of the cell suspension from the first container 310.

The control device 350 determines the amount of cell suspension to be seeded and the amount of diluent required to adjust the cell suspension to be seeded to a predetermined living cell concentration on the basis of the living cell concentration and the amount data of the cell suspension.

The control device 350 feeds the determined amount of cell suspension from the first container 310 to the mixing unit 330 and then closes the valve 362 to stop the pump 372.

In addition, the control device 350 opens the valve 363 to drive the pump 373 such that the determined amount of diluent is fed from the second container 320 to the mixing unit 330 and then closes the valve 363 to stop the pump 373.

As described above, the living cell concentration can be adjusted more rapidly by feeding the cell dispersion to the mixing unit 330 while measuring the living cell concentration.

As in Cell Culture Example 1, after the cell suspension is diluted by the mixing unit 330, the diluted cell suspension is seeded in the culture container 340.

### (Embodiment 2)

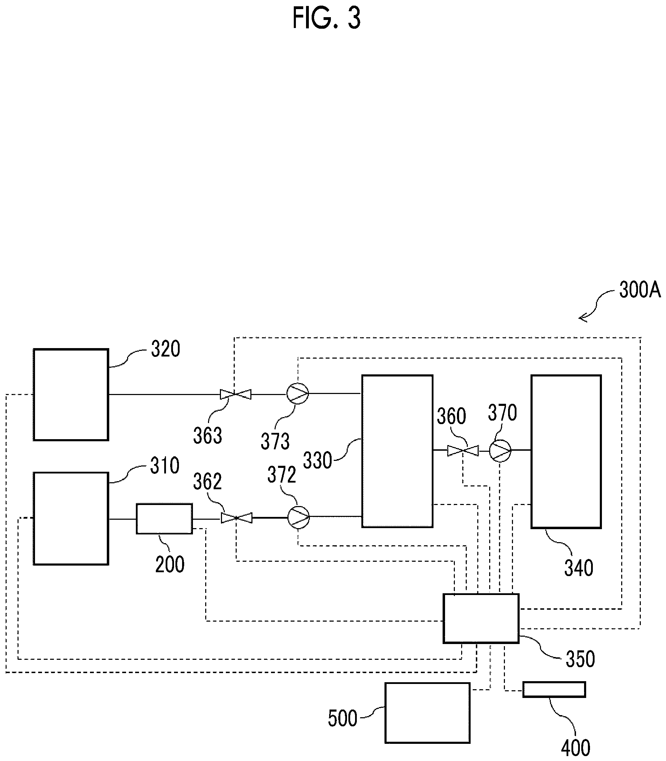

In another embodiment, a cell culture apparatus 300A differs from the cell culture apparatus 300 in that the living cell concentration measurement device 200 is disposed in the flow path between the first container 310 and the mixing unit 330 as illustrated in Fig. 3.

The following Cell Culture Examples 3 and 4 will be described using the cell culture apparatus 300A as an example.

### -Cell Culture Example 3-

The control device 350 opens the valve 362 to drive the pump 372 such that the cell suspension is fed from the first container 310 to the living cell concentration measurement device 200 and then closes the valve 362 to stop the pump 372.

After the living cell concentration of the cell suspension is measured by the living cell concentration measurement device 200, the control device 350 acquires the living cell concentration of the cell suspension from the living cell concentration measurement device 200 and acquires the amount data of the cell suspension from the first container 310.

The control device 350 determines the amount of cell suspension to be seeded and the amount of diluent required to adjust the cell suspension to be seeded to a predetermined living cell concentration on the basis of the living cell concentration and the amount data of the cell suspension.

The control device 350 opens the valve 362 to drive the pump 372 such that the determined amount of cell suspension is fed from the first container 310 to the mixing unit 330 and then closes the valve 362 to stop the pump 372.

In addition, the control device 350 opens the valve 363 to drive the pump 373 such that the determined amount of diluent is fed from the second container 320 to the mixing unit 330 and then closes the valve 363 to stop the pump 373.

As in Cell Culture Example 1, after the cell suspension is diluted by the mixing unit 330, the diluted cell suspension is seeded in the culture container 340.

### -Cell Culture Example 4-

The control device 350 opens the valve 362 to drive the pump 372 such that the cell suspension is fed from the first container 310 to the living cell concentration measurement device 200 and then starts the feeding of the cell suspension from the first container 310 to the mixing unit 330, without closing the valve 362 to stop the pump 372.

The living cell concentration of the cell suspension is measured by the living cell concentration measurement device 200 for at least a portion of the liquid feeding time until the cell suspension reaches the mixing unit 330 from the first container 310.

The control device 350 acquires the living cell concentration of the cell suspension from the living cell concentration measurement device 200 and acquires the amount data of the cell suspension from the first container 310.

The control device 350 determines the amount of cell suspension to be seeded and the amount of diluent required to adjust the cell suspension to be seeded to a predetermined living cell concentration on the basis of the living cell concentration and the amount data of the cell suspension.

The control device 350 feeds the determined amount of cell suspension from the first container 310 to the mixing unit 330 and then closes the valve 362 to stop the pump 372.

In addition, the control device 350 opens the valve 363 to drive the pump 373 such that the determined amount of diluent is fed from the second container 320 to the mixing unit 330 and then closes the valve 363 to stop the pump 373.

As described above, the living cell concentration can be adjusted more rapidly by feeding the cell dispersion to the mixing unit 330 while measuring the living cell concentration.

As in Cell Culture Example 1, after the cell suspension is diluted by the mixing unit 330, the diluted cell suspension is seeded in the culture container 340.

### (Embodiment 3)

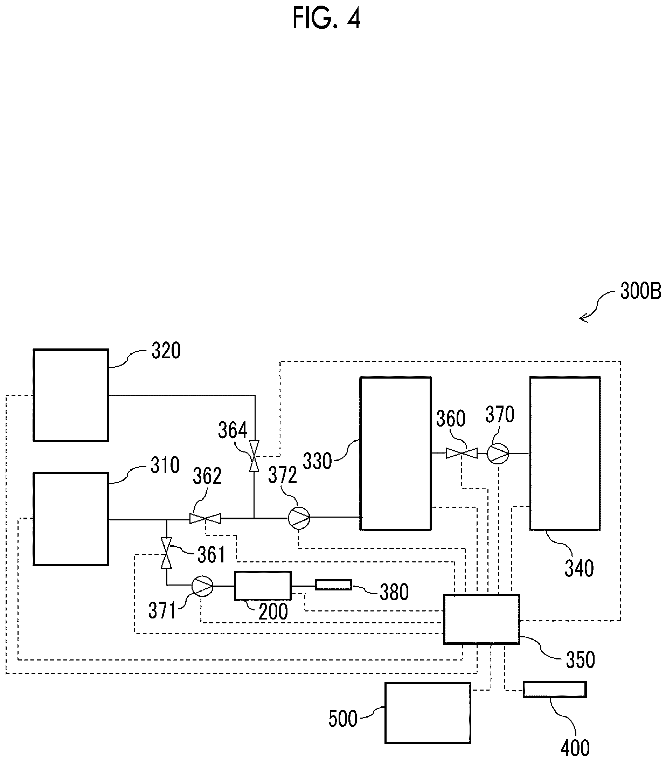

In another embodiment, a cell culture apparatus 300B differs from the cell culture apparatus 300 in that a single pump 372 is disposed between the first container 310 and the mixing unit 330 and between the second container 320 and the mixing unit 330 as illustrated in Fig. 4.

The following Cell Culture Example 5 will be described using the cell culture apparatus 300B as an example.

### -Cell Culture Example 5-

The control device 350 opens the valve 361 to drive the pump 371 such that the cell suspension is fed from the first container 310 to the living cell concentration measurement device 200 and then closes the valve 361 to stop the pump 371.

The living cell concentration of the cell suspension is measured by the living cell concentration measurement device 200, and the cell suspension that has been used for the measurement is discharged to the waste liquid container 380.

The control device 350 acquires the living cell concentration of the cell suspension from the living cell concentration measurement device 200 and acquires the amount data of the cell suspension from the first container 310.

The control device 350 determines the amount of cell suspension to be seeded and the amount of diluent required to adjust the cell suspension to be seeded to a predetermined living cell concentration on the basis of the living cell concentration and the amount data of the cell suspension.

The control device 350 opens the valve 362 and a valve 364 to drive the pump 372 such that the determined amount of diluent is fed from the second container 320 to the mixing unit 330 at the same time as the determined amount of cell suspension is fed from the first container 310 to the mixing unit 330. Then, the control device 350 closes the valve 362 and the valve 364 to stop the pump 372.

As in Cell Culture Example 1, after the cell suspension is diluted by the mixing unit 330, the diluted cell suspension is seeded in the culture container 340.

### -Cell Culture Example 6-

The control device 350 opens the valve 362 and the valve 364 to drive the pump 372 at the same time as opening the valve 361 to drive the pump 371. Then, the control device 350 starts the feeding of the cell suspension from the first container 310 to the mixing unit 330 while feeding the cell suspension from the first container 310 to the living cell concentration measurement device 200 and starts the feeding of the diluent from the second container 320 to the mixing unit 330. After feeding the cell suspension to the living cell concentration measurement device 200, the control device 350 closes the valve 361 to stop the pump 371.

The living cell concentration of the cell suspension is measured by the living cell concentration measurement device 200 for at least a portion of the liquid feeding time until the cell suspension reaches the mixing unit 330 from the first container 310. The cell suspension that has been used for the measurement is discharged to the waste liquid container 380.

The control device 350 acquires the living cell concentration of the cell suspension from the living cell concentration measurement device 200 and acquires the amount data of the cell suspension from the first container 310.

The control device 350 determines the amount of cell suspension to be seeded and the amount of diluent required to adjust the cell suspension to be seeded to a predetermined living cell concentration on the basis of the living cell concentration and the amount data of the cell suspension.

The control device 350 feeds the determined amount of cell suspension from the first container 310 to the mixing unit 330, feeds the determined amount of diluent from the second container 320 to the mixing unit 330, and then closes the valve 362 and the valve 340 to stop the pump 372.

As described above, the living cell concentration can be adjusted more rapidly by feeding the cell dispersion to the mixing unit 330 while measuring the living cell concentration.

As in Cell Culture Example 1, after the cell suspension is diluted by the mixing unit 330, the diluted cell suspension is seeded in the culture container 340.

### (Embodiment 4)

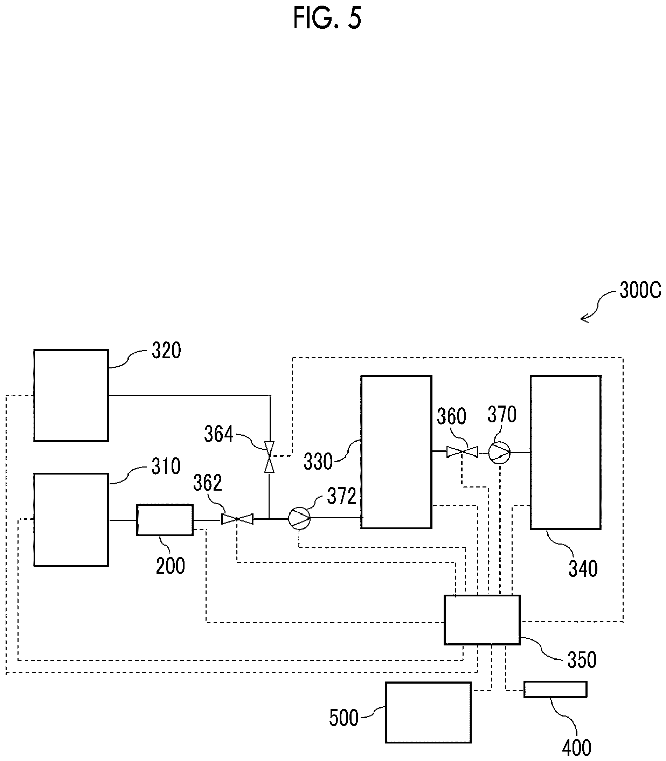

In another embodiment, a cell culture apparatus 300C differs from the cell culture apparatus 300 in that the living cell concentration measurement device 200 is disposed in the flow path between the first container 310 and the mixing unit 330 as illustrated in Fig. 5. In addition, a single pump 372 is disposed between the first container 310 and the mixing unit 330 and between the second container 320 and the mixing unit 330.

The following Cell Culture Examples 7 and 8 will be described using the cell culture apparatus 300C as an example.

### -Cell Culture Example 7-

The control device 350 opens the valve 362 to drive the pump 372 such that the cell suspension is fed from the first container 310 to the living cell concentration measurement device 200 and then closes the valve 362 to stop the pump 372.

After the living cell concentration of the cell suspension is measured by the living cell concentration measurement device 200, the control device 350 acquires the living cell concentration of the cell suspension from the living cell concentration measurement device 200 and acquires the amount data of the cell suspension from the first container 310.

The control device 350 determines the amount of cell suspension to be seeded and the amount of diluent required to adjust the cell suspension to be seeded to a predetermined living cell concentration on the basis of the living cell concentration and the amount data of the cell suspension.

The control device 350 opens the valve 362 and a valve 364 to drive the pump 372 such that the determined amount of diluent is fed from the second container 320 to the mixing unit 330 at the same time as the determined amount of cell suspension is fed from the first container 310 to the mixing unit 330. Then, the control device 350 closes the valve 362 and the valve 364 to stop the pump 372.

As in Cell Culture Example 1, after the cell suspension is diluted by the mixing unit 330, the diluted cell suspension is seeded in the culture container 340.

### -Cell Culture Example 8-

The control device 350 opens the valve 362 to drive the pump 372 such that the cell suspension is fed from the first container 310 to the living cell concentration measurement device 200 and then starts the feeding of the cell suspension from the first container 310 to the mixing unit 330, without closing the valve 362 to stop the pump 372.

The living cell concentration of the cell suspension is measured by the living cell concentration measurement device 200 for at least a portion of the liquid feeding time until the cell suspension reaches the mixing unit 330 from the first container 310.

The control device 350 acquires the living cell concentration of the cell suspension from the living cell concentration measurement device 200 and acquires the amount data of the cell suspension from the first container 310.

The control device 350 determines the amount of cell suspension to be seeded and the amount of diluent required to adjust the cell suspension to be seeded to a predetermined living cell concentration on the basis of the living cell concentration and the amount data of the cell suspension.

The control device 350 opens the valve 364 to feed the determined amount of diluent from the second container 320 to the mixing unit 330 at the same time as feeding the determined amount of cell suspension from the first container 310 to the mixing unit 330. Then, the control device 350 closes the valve 362 and the valve 364 to stop the pump 372.

As described above, the living cell concentration can be adjusted more rapidly by feeding the cell dispersion to the mixing unit 330 while measuring the living cell concentration.

As in Cell Culture Example 1, after the cell suspension is diluted by the mixing unit 330, the diluted cell suspension is seeded in the culture container 340.

### (Embodiment 5)

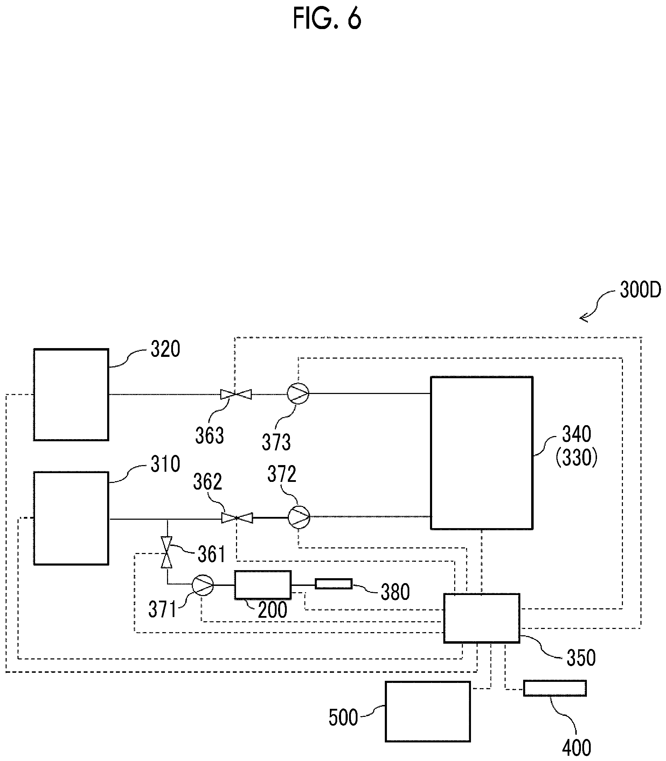

In another embodiment, unlike the cell culture apparatus 300, a cell culture apparatus 300D is a suspension culture apparatus in which the culture container 340 also serves as the mixing unit 330 as illustrated in Fig. 6. The culture container 340 comprises a circulation mechanism (not illustrated) that can circulate the diluted cell suspension.

The following Cell Culture Examples 9 and 10 will be described using the cell culture apparatus 300D as an example.

### -Cell Culture Example 9-

As in Cell Culture Example 1, the living cell concentration is measured by the living cell concentration measurement device 200, and the amounts of cell suspension and diluent are determined. Then, the determined amounts of cell suspension and diluent are fed to the culture container 340 (mixing unit 330).

The control device 350 drives the circulation mechanism comprised in the culture container 340 to seed the diluted cell suspension while mixing the cell suspension and the diluent.

### -Cell Culture Example 10-

As in Cell Culture Example 2, the living cell concentration is measured by the living cell concentration measurement device 200, and the amounts of cell suspension and diluent are determined. Then, the determined amounts of cell suspension and diluent are fed to the culture container 340 (mixing unit 330).

The control device 350 drives the circulation mechanism comprised in the culture container 340 to seed the diluted cell suspension while mixing the cell suspension and the diluent.

### (Embodiment 6)

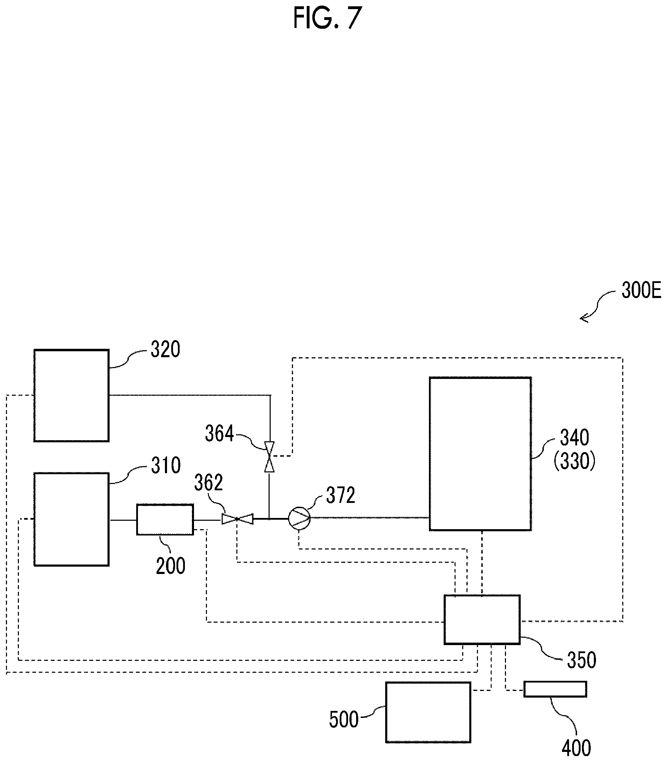

In another embodiment, a cell culture apparatus 300E differs from the cell culture apparatus 300A in that the culture container 340 also serves as the mixing unit 330 and comprises a shaking mechanism (not illustrated) that can shake the culture container 340 as illustrated in Fig. 7.

The following Cell Culture Examples 11 and 12 will be described using the cell culture apparatus 300E as an example.

### -Cell Culture Example 11-

As in Cell Culture Example 3, the living cell concentration is measured by the living cell concentration measurement device 200, and the amounts of cell suspension and diluent are determined. Then, the determined amounts of cell suspension and diluent are fed to the culture container 340 (mixing unit 330).

The control device 350 drives the shaking mechanism comprised in the culture container 340 to seed the diluted cell suspension while mixing the cell suspension and the diluent.

### -Cell Culture Example 12-

As in Cell Culture Example 4, the living cell concentration is measured by the living cell concentration measurement device 200, and the amounts of cell suspension and diluent are determined. Then, the determined amount of cell suspension and diluent are fed to the culture container 340 (mixing unit 330).

The control device 350 drives the shaking mechanism comprised in the culture container 340 to seed the diluted cell suspension while mixing the cell suspension and the diluent.

### (Embodiment 7)

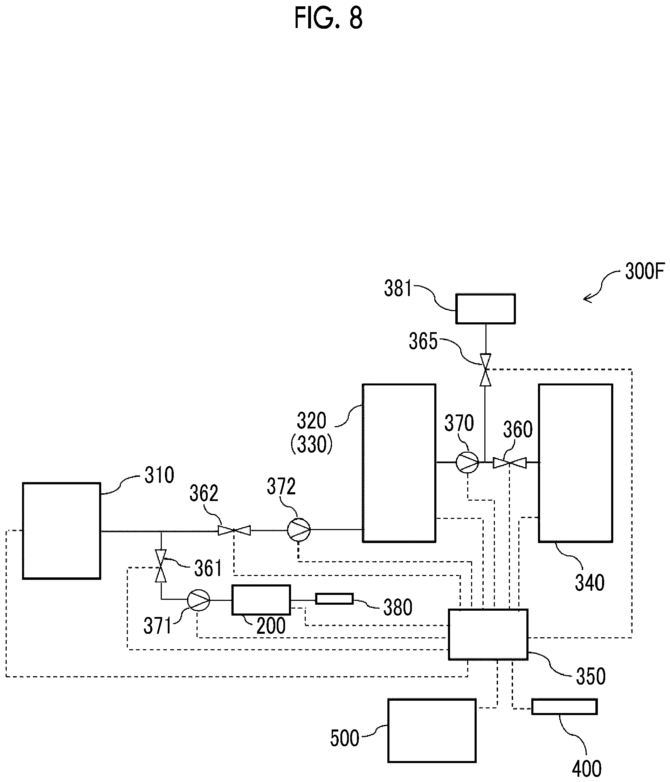

In another embodiment, a cell culture apparatus 300F differs from the cell culture apparatus 300 in that the second container 320 also serves as the mixing unit 330 as illustrated in Fig. 8. The second container 320 comprises a mixing mechanism (not illustrated).

The following Cell Culture Example 13 will be described using the cell culture apparatus 300F as an example.

### -Cell Culture Example 13-

As in Cell Culture Example 1, the living cell concentration is measured by the living cell concentration measurement device 200, and the amounts of cell suspension and diluent are determined.

In a case in which the amount of diluent held in the second container 320 is larger than the determined amount, the control device 350 opens a valve 365 to drive the pump 370 such that the excess diluent is fed to a waste liquid container 381 and then closes the valve 365 to stop the pump 370. Then, the determined amount of diluent remains in the second container 320.

The control device 350 feeds the determined amount of cell suspension to the second container 320 (mixing unit 330). The mixing mechanism (not illustrated) comprised in the second container 320 is driven to mix the cell suspension and the diluent such that the cell suspension is diluted.

The control device 350 opens the valve 360 to drive the pump 370 such that the diluted cell suspension is fed from the second container 320 to the culture container 340 and seeded in the culture container 340.

### (Embodiment 8)

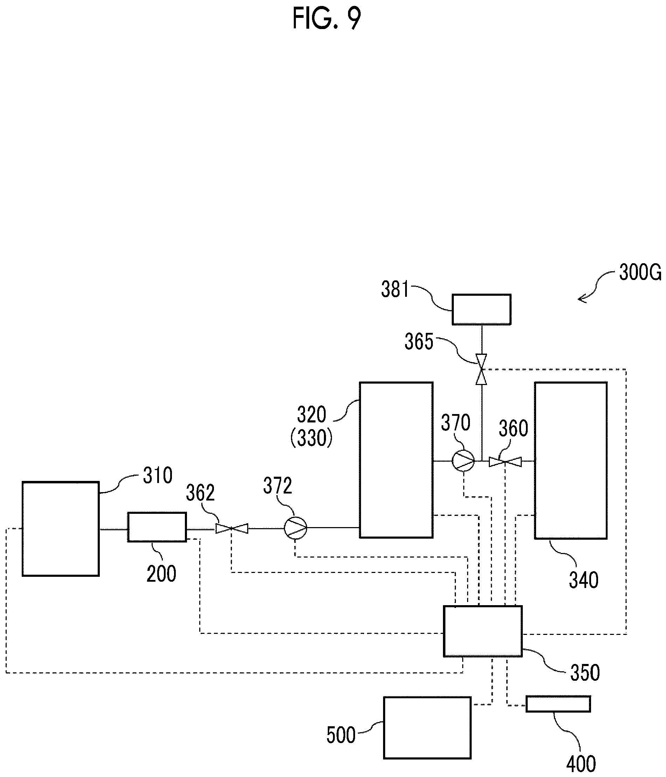

In another embodiment, a cell culture apparatus 300G differs from the cell culture apparatus 300A in that the second container 320 also serves as the mixing unit 330 as illustrated in Fig. 9. The second container 320 comprises a mixing mechanism (not illustrated).

The following Cell Culture Example 14 will be described using the cell culture apparatus 300G as an example.

### -Cell Culture Example 14-

As in Cell Culture Example 3, the living cell concentration is measured by the living cell concentration measurement device 200, and the amounts of cell suspension and diluent are determined.

In a case in which the amount of diluent held in the second container 320 is larger than the determined amount, the control device 350 opens the valve 365 to drive the pump 370 such that the excess diluent is fed to the waste liquid container 381 and then closes the valve 365 to stop the pump 370. Then, the determined amount of diluent remains in the second container 320.

The control device 350 feeds the determined amount of cell suspension to the second container 320 (mixing unit 330). The mixing mechanism (not illustrated) comprised in the second container 320 is driven to mix the cell suspension and the diluent such that the cell suspension is diluted.

The control device 350 opens the valve 360 to drive the pump 370 such that the diluted cell suspension is fed from the second container 320 to the culture container 340 and seeded in the culture container 340.

In Embodiments 1, 3, 5, and 7 (Figs. 1, 4, 6, and 8), the cell suspension used to measure the living cell concentration is fed from the first container 310 to the living cell concentration measurement device 200 by the pump 371. Then, the cell suspension that has been used for the measurement is discharged to the waste liquid container 380.

In another embodiment, a syringe is used as the waste liquid container 380 and is driven by a syringe pump (not illustrated) to feed the cell suspension to the living cell concentration measurement device 200, and the cell suspension that has been used for the measurement can be collected.

As described above, the cell culture apparatus according to the present disclosure can rapidly adjust the living cell concentration of the target cells.

### <Living Cell Concentration Adjustment Method>

A living cell concentration adjustment method according to the present disclosure comprises: a step of individually determining whether a plurality of target cells in a cell suspension are alive or dead to measure a living cell concentration of the target cells in the cell suspension (hereinafter, sometimes referred to as a "living cell concentration measurement step"); a step of determining an amount of the cell suspension to be seeded and an amount of the diluent required to adjust the cell suspension to be seeded to a predetermined living cell concentration, on the basis of the living cell concentration and amount data of the cell suspension (hereinafter, sometimes referred to as a "liquid amount determination step"); and a step of mixing the amount of the cell suspension and the amount of the diluent (hereinafter, sometimes referred to as a "diluting step").

Hereinafter, each step will be described in detail.

### [Living Cell Concentration Measurement Step]

In the living cell concentration measurement step, it is individually determined whether the plurality of target cells in the cell suspension are alive or dead to measure the living cell concentration of the target cells in the cell suspension.

The method for measuring the living cell concentration is not particularly limited.

For example, a step of analyzing a connected image obtained from a plurality of images of the target cell to determine whether the target cell is alive or dead and measuring the living cell concentration (hereinafter, sometimes referred to as a "living cell concentration measurement step according to Aspect 1") is given as an example, which will be described in detail below.

In addition, a step of analyzing a phase image of the target cell captured by the digital holographic microscope to determine whether the target cell is alive or dead and measuring the living cell concentration (hereinafter, sometimes referred to as a "living cell concentration measurement step according to Aspect 2") is given as an example.

Hereinafter, the living cell concentration measurement steps according to Aspects 1 and 2 will be specifically described.

### (Living Cell Concentration Measurement Step According to Aspect 1)

The living cell concentration measurement step according to Aspect 1 includes: a step of acquiring images of the target cell captured in a plurality of focal planes including an in-focus plane of the target cell in a direction opposite to a side on which the target cell is irradiated with the light (hereinafter, sometimes referred to as an "image acquisition step"); a step of acquiring an image piece including a central portion and an outer peripheral portion of the target cell from each of the images (hereinafter, sometimes referred to as an "image piece acquisition step"); a step of connecting the image pieces in an order of an imaging direction of the focal plane to create a connected image for analysis (hereinafter, sometimes referred to as a "connected-image-for-analysis creation step"); a step of extracting a feature amount from the connected image for analysis (hereinafter, sometimes referred to as a "feature amount extraction step"); a step of determining whether the plurality of target cells in the cell suspension are alive or dead on the basis of the feature amount of the connected image for analysis and a predetermined range of the feature amount (hereinafter, sometimes referred to as a "life-and-death determination step"); and a step of determining the living cell concentration of the target cells in the cell suspension on the basis of a result of determining whether the target cells are alive or dead (hereinafter, sometimes referred to as a "living cell concentration determination step").

Unless otherwise specified, conditions for creating the connected image for analysis (for example, imaging conditions) are the same as the conditions for creating the connected image for reference at least to the extent that it can be determined whether the target cells are alive or dead and the target cells can be identified. In addition, even in a case in which different connected images for analysis are compared with each other, the conditions for creating these connected images for analysis are the same at least to the extent that the comparison is possible in the determination of whether the target cells are alive or dead and the identification of the target cells.

### -Image Acquisition Step-

In the image acquisition step, the image of the target cell captured in the plurality of focal planes including the in-focus plane of the target cell or the focal plane in the vicinity of the in-focus plane in the direction opposite to the side on which the target cell is irradiated with light is acquired.

The image of the target cell can be acquired by imaging the cell suspension with the imaging device.

The unit for changing the focal plane is not particularly limited, and a method for changing the distance between the target cell and the imaging device is given as an example. In addition, an example of a method of performing imaging while keeping the distance between the target cell and the imaging device constant is a method for changing the focus of a liquid lens using an imaging device provided with the liquid lens. The focal plane is moved with a change in focal length.

For example, as illustrated in Fig. 10, the holding container 40 in which the target cell (cell suspension) C is accommodated may be disposed between the light source 10 and the imaging device 20, and the target cell may be imaged while the focal plane is being changed by moving the stage 30 on which the holding container 40 is placed.

In addition, a holding container in which cells (cell suspension) are accommodated may be disposed between the light source and the imaging device, and the cell may be imaged while the focal plane is being changed by moving the imaging device.

Further, a holding container in which cells (cell suspension) are accommodated may be disposed between the light source and the imaging device, and the cell may be imaged while the focal plane is being changed by changing the focus of the liquid lens provided in the imaging device, without moving the imaging device and the stage.

The target cell may be imaged while the focal plane is being moved from the light source 10 to the imaging device 20. Alternatively, the target cell may be imaged while the focal plane is being changed from the imaging device 20 to the light source 10.

The imaging device consists of a combination of an imaging lens and an area sensor. The imaging lens may be, for example, a telecentric lens or a microscope objective lens.

The type, aperture angle, magnification, and the like of the lens in the imaging device are not particularly limited, but may affect focusing and the divergence of light in the connected image. Therefore, they may be appropriately selected such that imaging can be performed appropriately.

As the magnification of the lens is higher, a visual field is narrower, and the amount of cell suspension that can be imaged per visual field is smaller. Therefore, from the viewpoint of measuring a larger amount of cell suspension, for example, the magnification is preferably about 2 to 4. In addition, as the aperture angle of the lens is narrower, the discrimination sensitivity of the focusing point tends to be higher.

For example, a charge-coupled device (CCD) or a complementary metal-oxide semiconductor (CMOS) can be used as the area sensor of the imaging device. For example, the resolution of the area sensor is preferably set such that one pixel is about 1 µm to 3 µm in consideration of the magnification of the lens.

The light source is not particularly limited, and an example of the light source is a light emitting diode (LED).

From the viewpoint of the lens effect, it is preferable that the light source emits parallel light.

The positions of the plurality of focal planes in which the target cell is imaged and the number of focal planes are not particularly limited as long as the plurality of focal planes include the in-focus plane of the target cell. However, it is preferable to set the positions of the focal planes and the number of focal planes such that focusing and the divergence of light are seen in the connected image.

For example, the target cell may be imaged while the focal plane is being moved over a wide range from the lower side to the upper side of the holding container, including from the bottom surface to the top surface of the holding container.

It is preferable that the distance between adjacent focal planes is constant. For example, in a case in which the stage 30 illustrated in Fig. 10 is used, the position of the stage 30 may be specified by an encoder, and the target cell may be imaged while the stage 30 is being moved at equal intervals.

It is preferable that the interval at which the focal plane is moved is, for example, about 0.01 mm. In addition, since the moving distance of the focal plane in the cell suspension is shorter than the moving distance of the stage due to the influence of the refractive index of the cell suspension, it is preferable to determine the moving distance of the stage in consideration of this.

For convenience, in a case in which the focal plane is changed, for example, an image may be acquired with a focus on the bottom surface (or the vicinity thereof) of the holding container that holds the target cell, and the bottom surface may be used as a reference plane.

In addition, since the connected image is obtained by defining the reference plane and moving the focal plane from the reference plane within a predetermined range, it is possible to create the connected image under the same conditions, which is useful for analyzing the connected image.

A commercially available counting cell, a blood cell counter, or a measurement cell having a structure similar to these can be used as the holding container. For example, a holding container may be used in which a liquid thickness is about 0.1 mm and a cell concentration is about 1 × 10⁶ cells/ml in a case in which the cell suspension is accommodated in the holding container and the liquid thickness increases in a case in which the cell concentration is further reduced.

For example, in a case in which the cell suspension includes other substances (for example, other cells, red blood cells, and oil droplets), a substance that has a diameter within a predetermined range and has a shape circularity within a predetermined range may be selected in advance by image processing using image processing software. The effect of shortening the total time required to determine whether the cell is alive or dead can be obtained by excluding a substance that is clearly different from the target cell.

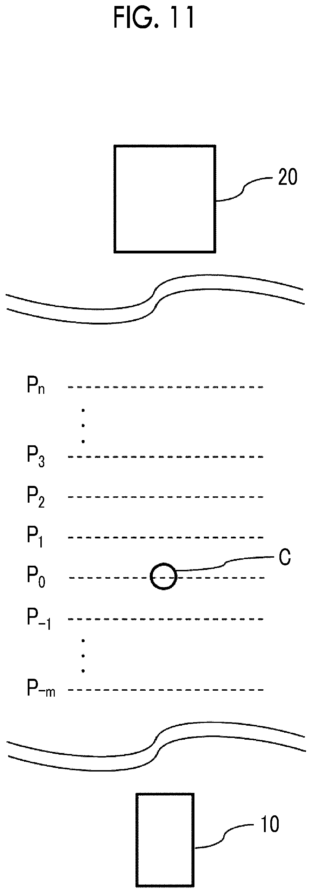

For example, as illustrated in Fig. 11, a plurality of images may be acquired by irradiating the target cell C with light and imaging the target cell C in a plurality of focal planes Pₙ to P₋ₘ including an in-focus plane P₀ of the target cell C in a direction opposite to the side on which the target cell C is irradiated with light while changing the focal plane.

The focal plane Pₙ is an n-th focal plane from the in-focus plane P₀ of the target cell C in the direction of the imaging device 20. The focal plane P₋ₘ is an m-th focal plane from the in-focus plane P₀ of the target cell C in the direction of the light source 10. At least one of m or n may be an integer equal to or greater than 1, and m + n may be an integer equal to or greater than 1. Fig. 11 illustrates an example in which m is equal to or greater than 2 and n is equal to or greater than 4.

Hereinafter, an example of a case in which the target cell C is the living cell will be described.

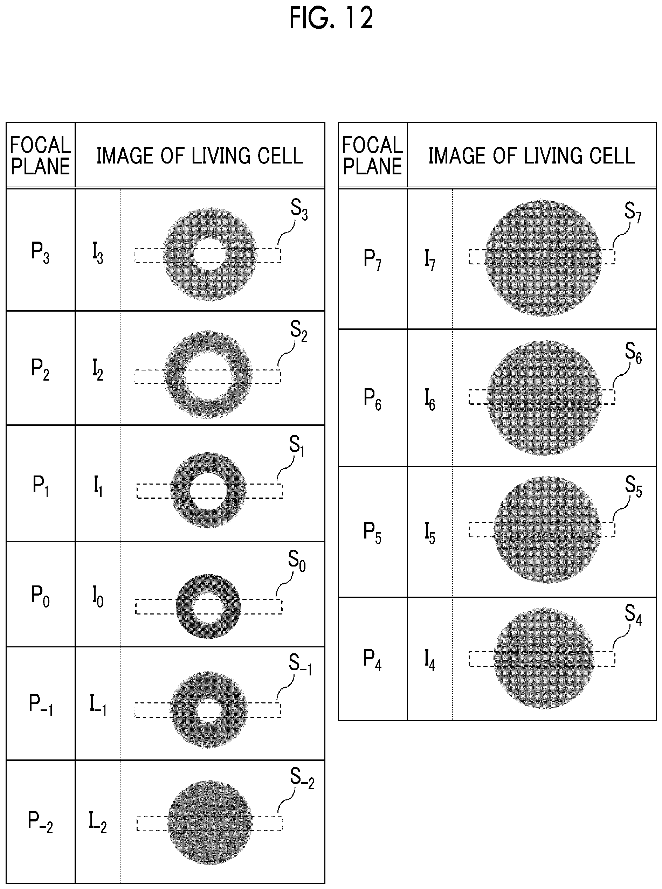

In a case in which the target cell C is the living cell, for example, images I₇ to I₋₂ of the living cell are obtained by imaging the living cell in a plurality of focal planes P₇ to P₋₂ including the in-focus plane P₀ of the living cell while changing the focal plane as illustrated in Fig. 12.

The image I₀ is captured in the in-focus plane P₀ of the living cell, and the contour of the living cell is clear. Further, the focusing point of the lens effect whose contour is unclear is present in a central portion of the living cell.

The image I₁ is captured in the first focal plane P₁ from the in-focus plane P₀ of the living cell in the direction of the imaging device 20, and the contour of the living cell is unclear. In addition, the focusing point of the lens effect is present in the central portion of the living cell, and the contour of the focusing point is clear. That is, the focal plane P₁ is an example of the in-focus plane of the focusing point of the lens effect.

The image I₂ and the image I₃ are captured in the second focal plane P₂ and the third focal plane P₃ from the in-focus plane P₀ of the living cell in the direction of the imaging device 20, respectively, and the contour of the living cell is unclear. Further, the focusing point of the lens effect whose contour is unclear is present in the central portion of the living cell.

The images I₄ to I₇ are captured in the fourth to seventh focal planes P₄ to P₇ from the in-focus plane P₀ of the living cell in the direction of the imaging device 20, respectively, and the contour of the living cell is unclear. In addition, the focusing point of the lens effect is not seen.

The image I₋₁ is captured in the first focal plane P₋₁ from the in-focus plane P₀ of the living cell in the direction of the light source 10, and the contour of the living cell is unclear. Further, the focusing point of the lens effect whose contour is unclear is present in the central portion of the living cell.

The image I₋₂ is captured in the second focal plane P₋₂ from the in-focus plane P₀ of the living cell in the direction of the light source 10, and the contour of the living cell is unclear. In addition, the focusing point of the lens effect is not seen.

As illustrated in, for example, the images I₇ to I₋₂, the contour of the living cell tends to be larger as the focal plane is farther from the in-focus plane P₀ of the living cell.

On the side (that is, the side of the imaging device 20) on which the focusing point of the lens effect is formed, the size of the focusing point of the lens effect tends to be larger as the focal plane is farther from the in-focus plane P₁ of the focusing point of the lens effect, for example, as shown in the image I₁ and the image I₂. However, in the focal plane P₃ that is far from the in-focus plane P₁ of the focusing point of the lens effect, the size of the focusing point of the lens effect is small as shown in the image I₃. In the focal plane P₄ that is further from the in-focus plane Pi, the focusing point is not seen.

In addition, on a side (that is, the side of the light source 10) opposite to the side on which the focusing point of the lens effect is formed, the size of the focusing point of the lens effect tends to be smaller as the focal plane is farther from the in-focus plane P₁ of the focusing point of the lens effect, for example, as shown in the image I₋₁. Further, in the focal plane P₋₂ that is further from the in-focus plane Pi, the focusing point of the lens effect is not seen.

Next, an example of a case in which the target cell is the dead cell will be described.

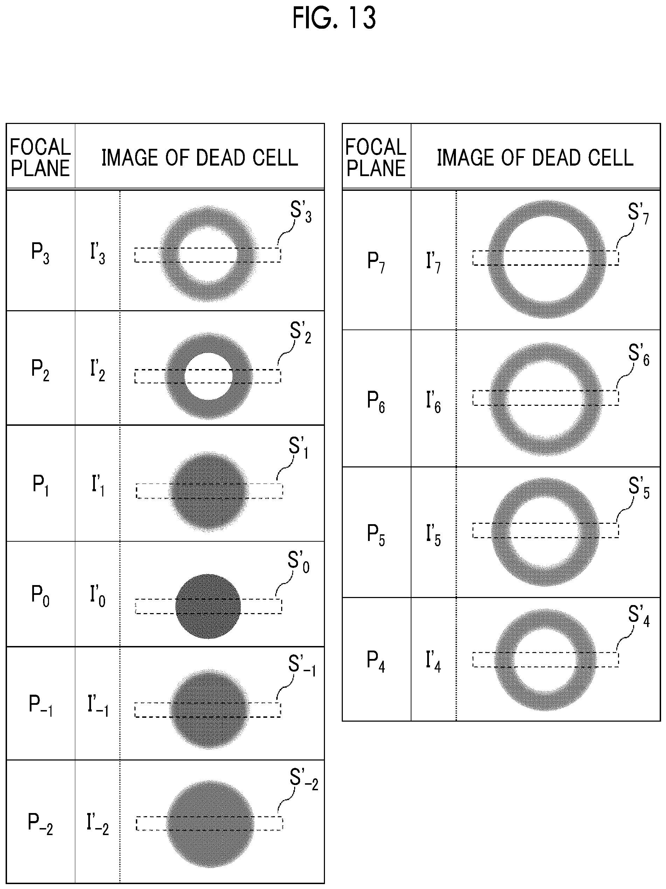

In a case in which the cell is the dead cell, for example, images I'₇ to I'₋₂ of the dead cell are obtained by imaging the dead cell in a plurality of focal planes P₇ to P₋₂ including an in-focus plane P₀ of the dead cell while changing the focal plane as illustrated in Fig. 13.

The image I'₀ is captured in the in-focus plane P₀ of the dead cell, and the contour of the dead cell is clear.

The image I'₁ is captured in the first focal plane P₁ from the in-focus plane P₀ of the dead cell in the direction of the imaging device 20, and the contour of the dead cell is unclear.

The image I'₂ is captured in the second focal plane P₂ from the in-focus plane P₀ of the dead cell in the direction of the imaging device 20, and the contour of the dead cell is unclear. In addition, the dead cell is opaque as compared to the living cell because the cell membrane is broken and light is scattered on the outer peripheral surface of the dead cell. As a result, in a case in which the dead cell is irradiated with light, a phenomenon similar to the diffraction of plane waves of light caused by a shielding material occurs, and a bright portion is formed in the vicinity of the center of the dead cell in the direction opposite to the side on which the dead cell is irradiated with light. This is a result of overlapping diffracted light components (hereinafter, the above-described portion is referred to as a "focusing point of diffracted light"). The focusing point of the diffracted light is present in the central portion of the dead cell in the image I'₂, and the contour of the focusing point is clear. That is, the focal plane P₂ is an example of the in-focus plane of the focusing point of the diffracted light.

The images I'₃ to I'₇ are captured in the third to seventh focal planes P₃ to P₇ from the in-focus plane P₀ of the dead cell in the direction of the imaging device 20, respectively, and the contour of the dead cell is unclear. In addition, the focusing point of the diffracted light whose contour is unclear is present in the central portion of the dead cell.

The images I'₋₁ and I'₋₂ are captured in the first and second focal planes P₋₁ and P₋₂ from the in-focus plane P₀ of the dead cell in the direction of the light source 10, respectively, and the contour of the dead cell is unclear.

Similarly to the living cell, the contour of the dead cell tends to be larger as the focal plane is farther from the in-focus plane P₀ of the dead cell, for example, as shown in the images I₇ to I₋₂.

The size of the focusing point of the diffracted light tends to be larger as the focal plane is further from the in-focus plane P₂ of the focusing point of the diffracted light, for example, as shown in the images I'₇ to I'₂.

The above is an example described with reference to Figs. 12 and 13, and the contour of the target cell and the appearance of the focusing point may differ depending on, for example, the sphericity and size of the target cell.

In some cases, the focusing point of the lens effect and the focusing point of the diffracted light are simply referred to as a "focusing point".

### -Image Piece Acquisition Step-

In the image piece acquisition step, an image piece including the central portion and the outer peripheral portion of the target cell is acquired from each of the images obtained in the image acquisition step.

The image piece acquisition step may be performed by, for example, image processing using image processing software.

The position where the image piece is acquired from the image of the target cell is not particularly limited as long as the image piece includes the central portion and the outer peripheral portion of the target cell.

It is preferable that the image piece includes a portion in which the length of the target cell has the maximum value (that is, a portion in which the length of a straight line connecting any two points on the contour of the target cell has the maximum value).

For example, as illustrated in Fig. 12, image pieces S₇ to S₋₂ including the portion in which the length of the living cell has the maximum value may be acquired, or image pieces S'₇ to S'₋₂ including the portion in which the length of the dead cell has the maximum value may be acquired.

In addition, preprocessing may be performed on each image before the image piece is acquired from each image of the target cell. An example of the preprocessing is a process that sets concentric circles, which have the central portion of the target cell as their centers and have different radii, in units of pixels, calculates an average value of brightness on the circumference of each concentric circle, and draws a concentric circle whose brightness on the circumference has the average value (that is, a concentric circle whose brightness on the circumference is averaged and which has the same radius as the original concentric circle) to reconstruct the image of the target cell. This makes it possible to obtain symmetrical images that have the central portion of the target cell as their centers. Therefore, in a case in which the image piece is acquired, it is possible to remove the influence of a direction in which the image piece is cut out.

The use of the image piece acquired from the image subjected to the preprocessing makes it possible to obtain symmetric connected images (for example, the bilaterally symmetric connected images illustrated in Figs. 5 and 6) and makes it easier to extract the feature amount from the connected image in the subsequent feature amount extraction step.

### -Connected-Image-for-Analysis Creation Step-

In the connected-image-for-analysis creation step, each image piece obtained in the image piece acquisition step is connected in the order of the imaging direction of the focal plane to create a connected image for analysis.

The connected-image-for-analysis creation step may be performed by, for example, image processing using image processing software.

A method for connecting the image pieces is not particularly limited as long as the image pieces are connected in the order of the imaging direction of the focal plane. It is preferable to connect the image pieces along a straight line connecting the central portions of the target cell in each image piece such that the long sides of the image pieces are in contact with each other.

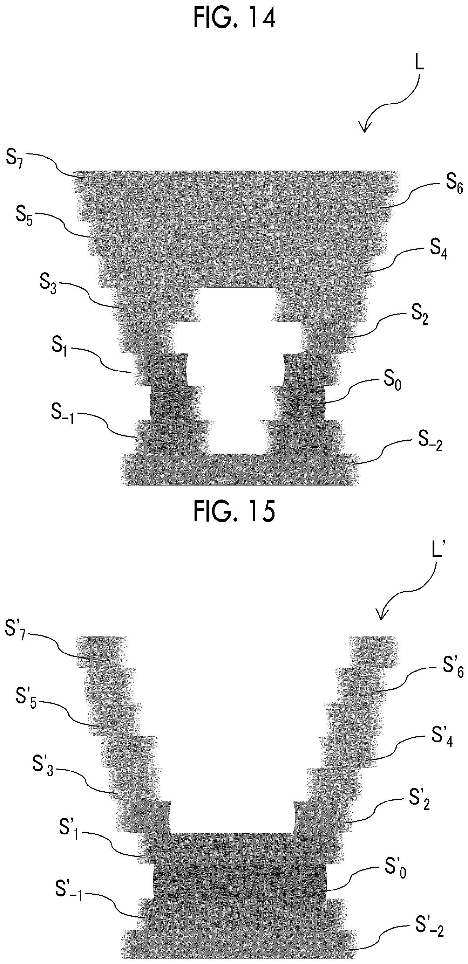

For example, as illustrated in Fig. 14, the image pieces S₇ to S₋₂ of the living cell acquired from Fig. 12 are connected in the order of the imaging direction of the focal plane along a line connecting the central portions of the living cell in each image piece to obtain a connected image L for analysis of the living cell.

In addition, for example, as illustrated in Fig. 15, the image pieces S'₇ to S'₋₂ of the dead cell acquired from Fig. 13 are connected in the order of the imaging direction of the focal plane along a line connecting the central portions of the dead cell in each image piece to obtain a connected image L' for analysis of the dead cell.

### -Feature Amount Extraction Step-

In the feature amount extraction step, the feature amount is extracted from the connected image for analysis obtained in the connected-image-for-analysis creation step.

The feature amount extraction step may be performed by, for example, image processing using image processing software.

### (Feature Amount Extracted from Connected Image)

The connected image includes various types of information related to the target cell. For example, the following feature amounts 1 to 11 can be obtained.

### -Feature Amount 1- (Determination of Whether Cell Is Alive or Dead Based on Whether Focusing Point of Lens Effect Is Present or Absent)

The connected image includes information related to whether the cell is alive or dead based on whether the focusing point of the lens effect is present or absent.

The connected image includes the image piece of the in-focus plane of the cell. In the in-focus plane of the cell, the living cell has the focusing point of the lens effect, but the dead cell does not have the focusing point of the lens effect.

For example, the connected image L of the living cell illustrated in Fig. 14 has the focusing point of the lens effect in the image piece S₀ of the in-focus plane P₀ of the living cell. On the other hand, for example, the connected image L' of the dead cell illustrated in Fig. 15 does not have the focusing point of the lens effect in the image piece S'₀ of the in-focus plane P₀ of the dead cell.

Therefore, it is possible to determine whether the cell is alive or dead on the basis of whether the focusing point of the lens effect is present or absent.

For example, the brightness of a central portion of the image piece of the in-focus plane of the cell may be set as the feature amount 1, and whether the cell is alive or dead may be determined on the basis of a predetermined range of the feature amount 1. Therefore, for the connected image for analysis, in a case in which the feature amount 1 is within the above-described range, it is possible to determine that the cell is the living cell.

In addition, in another embodiment, whether the cell is alive or dead may be determined on the basis of the feature amount 1 of the connected image for analysis, the feature amount 1 of the known connected image for reference of the target cell, and the result of determining whether or not the cell is the living cell.

### -Feature Amount 2- (Determination of Whether Cell Is Alive or Dead Based on Start Position of Focusing Point)

The connected image includes information related to whether the cell is alive or dead based on the start position of the focusing point.

The "start position of the focusing point" means the in-focus plane of the focusing point of the lens effect or the diffracted light.

The "start position of the focusing point" in the connected image means the image piece of the in-focus plane of the focusing point of the lens effect or the diffracted light.

In the living cell, the start position of the focusing point of the lens effect reflects the average refractive index of the cell.

In the case of the living cell, for example, as illustrated in Fig. 12, the in-focus plane P₁ of the focusing point of the lens effect is the start position of the focusing point of the lens effect.

On the other hand, in the case of the dead cell, for example, as illustrated in Fig. 13, the in-focus plane P₂ of the focusing point of the diffracted light is the start position of the focusing point of the diffracted light.

For example, as illustrated in Figs. 12 and 13, the start position of the focusing point of the lens effect is closer to the cell than the start position of the focusing point of the diffracted light. The reason is that, while the focusing point of the lens effect is formed by the focusing of the light transmitted through the living cell in the vicinity of the living cell, the focusing point of the diffracted light is formed by the focusing of the light passing through the vicinity of the outer peripheral surface of the dead cell at the position that is relatively far from the dead cell. That is, the focusing point of the lens effect formed in the living cell is formed closer to the cell than the focusing point of the diffracted light formed in the dead cell.

For example, in the connected image L of the living cell illustrated in Fig. 14, the image piece S₁ of the start position (in-focus plane Pi) of the focusing point of the lens effect is adjacent to the image piece S₀ of the in-focus plane P₀ of the living cell, and the start position of the focusing point of the lens effect is close to the in-focus plane P₀ of the living cell.

On the other hand, for example, in the connected image L' of the dead cell illustrated in Fig. 15, the image piece S'₂ of the start position (in-focus plane P₂) of the focusing point of the diffracted light is not adjacent to the image piece S'₀ of the in-focus plane P₀ of the dead cell, and the start position of the focusing point of the diffracted light is far from the in-focus plane P₀ of the dead cell.

Therefore, it is possible to determine whether the cell is alive or dead on the basis of the start position of the focusing point.

For example, the start position of the focusing point may be specified by the distance from the in-focus plane of the cell, and the feature amount 2 may be set. For example, a length between the image piece of the in-focus plane of the cell and the image piece of the start position of the focusing point (that is, the in-focus plane of the focusing point) in the direction in which the image pieces are connected may be set as the feature amount 2, and whether the target cell is alive or dead may be determined on the basis of a predetermined range of the feature amount 2. Therefore, for the connected image for analysis, in a case in which the feature amount 2 is within the above-described range, it is possible to determine that the cell is the living cell.

In addition, in another embodiment, whether the target cell is alive or dead may be determined on the basis of the feature amount 2 of the connected image for analysis, the feature amount 2 of the known connected image for reference of the target cell, and the result of determining whether or not the target cell is the living cell.

### -Feature amount 3- (Determination of Whether Cell Is Alive or Dead Based on Continuous Length of Focusing Point)

The connected image includes information related to whether the cell is alive or dead based on the continuous length of the focusing point.

The "continuous length of the focusing point" means a distance from the start position of the focusing point of the lens effect or the diffracted light to the focal plane in which the focusing point disappears.

In the connected image, the "continuous length of the focusing point" means the shortest length from the image piece of the in-focus plane of the focusing point of the lens effect or the diffracted light to the image piece of the focal plane in which the focusing point disappears.

In the living cell, the continuous length of the focusing point of the lens effect reflects the average refractive index of the cell.

In the case of the living cell, for example, as illustrated in Fig. 12, the distance from the start position (in-focus plane Pi) of the focusing point of the lens effect to the focal plane P₄ in which the focusing point of the lens effect disappears is the continuous length of the focusing point of the lens effect.

On the other hand, in the case of the dead cell, for example, as illustrated in Fig. 13, the distance from the start position (in-focus plane P₂) of the focusing point of the diffracted light to the focal plane in which the focusing point of the diffracted light disappears is the "continuous length of the focusing point" of the diffracted light. The focusing point continues up to the focal plane that is further away from the in-focus plane P₀ than the focal plane P₄, which is not illustrated in Fig. 13.

At the focusing point of the lens effect, light transmitted through a portion closer to the center of the living cell is focused at a position that is farther from the living cell, and light transmitted through a portion farther from the center of the living cell is focused at a position that is closer to the living cell. As a result, since the focusing points are connected according to a portion of the living cell through which light is transmitted, the focusing point of the lens effect has the continuous length of the focusing point. In addition, for the focusing point of the lens effect, the intensity of light decreases in a short distance due to divergence. Furthermore, in a case in which the living cell is irradiated with white light, a blue component (short-wavelength component) is focused at a position closer to the cell than a red component (long-wavelength component). Therefore, a difference in wavelength also contributes to the continuous length of the focusing point of the lens effect.

On the other hand, the focusing point of the diffracted light is formed by the focusing of light passing through the vicinity of the outer peripheral surface of the dead cell, and a change of an interference pattern depending on the distance from the dead cell extends over a relatively long distance. Therefore, the focusing point of the diffracted light has a longer continuous length than that of the lens effect. That is, the focusing point of the living cell has a shorter continuous length than the focusing point of the dead cell.

For example, in the connected image L of the living cell illustrated in Fig. 14, the focusing point of the lens effect is continuous from the image piece S₋₁ to the image piece S₃.

On the other hand, for example, in the connected image L' of the dead cell illustrated in Fig. 15, the focusing point of the diffracted light is continuous from the image piece S'₂ to the image piece S'₇.

As illustrated in Figs. 14 and 15, the continuous length of the focusing point in the connected image L of the living cell is shorter than the continuous length of the focusing point in the connected image L' of the dead cell.

Therefore, it is possible to determine whether the cell is alive or dead on the basis of the continuous length of the focusing point.

For example, in a region in which the focusing point is continuous, a length along a direction in which the image pieces are connected may be set as the feature amount 3, and whether the cell is alive or dead may be determined on the basis of a predetermined range of the feature amount 3. Therefore, for the connected image for analysis, in a case in which the feature amount 3 is within the above-described range, it is possible to determine that the cell is the living cell.

In addition, in another embodiment, whether the cell is alive or dead may be determined on the basis of the feature amount 3 of the connected image for analysis, the feature amount 3 of the known connected image for reference of the target cell, and the result of determining whether or not the cell is the living cell.

### -Feature Amount 4- (Identification of Cell Based on Start Position of Focusing Point)

The connected image includes information related to the identification of the cell based on the start position of the focusing point.

In the living cell, the start position of the focusing point of the lens effect reflects the average refractive index of the cell. For other substances (for example, other cells, red blood cells, and oil droplets) having an average refractive index different from that of the target cell, the start position of the focusing point is different from that of the target cell.

Therefore, it is possible to determine whether or not the cell to be determined for life or death is the target cell on the basis of the start position of the focusing point.

For example, the start position of the focusing point may be specified by the distance from the in-focus plane of the cell, and the feature amount 4 may be set. For example, the length between the image piece of the in-focus plane of the cell and the image piece of the start position of the focusing point (that is, the in-focus plane of the focusing point) in the direction in which the image pieces are connected may be set as the feature amount 4, and whether the cell is alive or dead may be determined on the basis of a predetermined range of the feature amount 4. Therefore, for the connected image for analysis, in a case in which the feature amount 4 is within the above-described range, it is possible to determine that the cell to be determined for life or death is the target cell.

In addition, in another embodiment, whether the cell is alive or dead may be determined on the basis of the feature amount 4 of the connected image for analysis, the feature amount 4 of the known connected image for reference of the target cell, and the result of determining whether or not the cell is the living cell.

The feature amount 4 may be the same as the feature amount 2. In addition, the feature amount 4 may be different from the feature amount 2. For example, the feature amount 4 may be set to be more appropriate for identifying the cell, and the feature amount 2 may be set to be more appropriate for determining whether the cell is alive or dead.

### -Feature Amount 5- (Identification of Cell Based on Continuous Length of Focusing Point)

The connected image includes information related to the identification of the cell based on the continuous length of the focusing point.

In the living cell, the continuous length of the focusing point of the lens effect reflects the average refractive index of the cell. Therefore, for other substances (for example, other cells, red blood cells, and oil droplets) having an average refractive index different from that of the target cell, the continuous length of the focusing point is different from that of the target cell.

Therefore, it is possible to determine whether or not the cell to be determined for life or death is the target cell on the basis of the continuous length of the focusing point.

For example, in a region in which the focusing point is continuous, a length along the direction in which the image pieces are connected may be set as the feature amount 5, and whether the cell is alive or dead may be determined on the basis of a predetermined range of the feature amount 5. Therefore, for the connected image for analysis, in the case in which the feature amount 5 is within the above-described range, it is possible to determine that the cell to be determined for life or death is the target cell.

In addition, in another embodiment, whether the cell is alive or dead may be determined on the basis of the feature amount 5 of the connected image for analysis, the feature amount 5 of the known connected image for reference of the target cell, and the result of determining whether or not the cell is the living cell.

The feature amount 5 may be the same as the feature amount 3. In addition, the feature amount 5 may be different from the feature amount 3. For example, the feature amount 5 may be set to be more appropriate for identifying the cell, and the feature amount 3 may be set to be more appropriate for determining whether the cell is alive or dead.

For the feature amount 4 and the feature amount 5, for example, unlike the living cell, the red blood cell has a shape like a concave lens. Therefore, in a case in which the red blood cell is irradiated with light, a focusing point is formed on a side opposite to the side on which the red blood cell is irradiated with light. In addition, a focusing point is also formed on the side on which the red blood cell is irradiated with light. In addition to the difference in the appearance of the focusing point, the red blood cell may differ from the living cell in the start position of the focusing point and the continuous length of the focusing point.

### -Feature Amount 6- (Identification of Cell Based on Size of Cell in In-Focus Plane)

The connected image may include information related to the identification of the cell based on the size of the cell in the in-focus plane.

The connected image includes the image piece of the in-focus plane of the cell. Therefore, in a case in which the image piece of the in-focus plane of the cell includes a portion in which the length of the cell has the maximum value (that is, a portion in which the length of a straight line connecting any two points on the contour of the cell has the maximum value), the maximum length of the portion can be used as the diameter of the cell. Then, for example, it is examined whether or not the diameter of the cell to be determined for life or death is the same as the known diameter of the target cell. In a case in which the diameters are not the same, it is possible to determine that the cell to be determined for life or death is not the target cell.

Therefore, it is possible to determine whether or not the cell to be determined for life or death is the target cell on the basis of the size of the cell in the in-focus plane.

For example, for the image piece of the in-focus plane of the cell to be determined for life or death, the length of a portion in which the length of the cell has the maximum value (that is, a portion in which the length of a straight line connecting any two points on the contour of the cell has the maximum value) may be set as the feature amount 6, and whether the cell is alive or dead may be determined on the basis of a predetermined range of the feature amount 6. Therefore, for the connected image for analysis, in the case in which the feature amount 6 is within the above-described range, it is possible to determine that the cell to be determined for life or death is the target cell.

In addition, in another embodiment, whether the cell is alive or dead may be determined on the basis of the feature amount 6 of the connected image for analysis, the feature amount 6 of the known connected image for reference of the target cell, and the result of determining whether or not the cell is the living cell.

### -Feature Amount 7- (Identification of Cell Based on Position of In-Focus Plane)

The connected image includes information related to the identification of the cell based on the position of the in-focus plane.

For example, in a cell suspension including the target cell and other substances (for example, other cells, red blood cells, and oil droplets), in a case in which the specific gravity of the cell is different from the specific gravity of other substances, there is a difference in how the cell and other substances sink (the degree of sinking) in the cell suspension, and the position of the cell is different from the positions of other substances in the depth direction of the cell suspension. Therefore, in a case in which the cell and other substance are imaged in the same focal plane to create a connected image, the position of the image piece of the in-focus plane of the cell is different from the positions of the image pieces of the in-focus planes of other substances (for example, the height in the direction in which the image pieces are connected). Then, for example, it is examined whether or not the position of the image piece of the in-focus plane of the cell to be determined for life or death is the same as the position of the image piece of the known in-focus plane of the target cell. In a case in which the positions are not the same, it is possible to determine that the cell to be determined for life or death is not the target cell.

Therefore, it is possible to determine whether or not the cell to be determined for life or death is the target cell on the basis of the position of the in-focus plane.

For example, a length between the image piece of the in-focus plane of the cell to be determined for life or death and the image piece of the lowermost focal plane in the direction in which the image pieces are connected may be set as the feature amount 7, and whether the cell is alive or dead may be determined on the basis of a predetermined range of the feature amount 7. Therefore, for the connected image for analysis, in the case in which the feature amount 7 is within the above-described range, it is possible to determine that the cell to be determined for life or death is the target cell.

In addition, in another embodiment, whether the cell is alive or dead may be determined on the basis of the feature amount 7 of the connected image for analysis, the feature amount 7 of the known connected image for reference of the target cell, and the result of determining whether or not the cell is the living cell.

### -Feature Amount 8- (Identification of Cell Based on Connected Shape of Focusing Points)

The connected image includes information related to the identification of the cell based on the connected shape of the focusing points.

The "connected shape of the focusing points" means the shape of a portion formed by connecting the focusing points in the connected image.

For example, for the connected image for analysis, an image of a portion in which the focusing points are connected may be set as the feature amount 8, and whether or not the cell to be determined for life or death is the target cell may be determined by a machine learning device on the basis of the feature amount 8 of the connected image for analysis, the feature amount 9 of the known connected image for reference of the target cell, and the result of determining whether or not the cell is the living cell.

The connected shape of the focusing points is related to the size of the focusing point in addition to the start position of the focusing point and the continuous length of the focusing point and reflects the average refractive index of the cell in the living cell. Therefore, other substances (for example, other cells, red blood cells, and oil droplets) having an average refractive index different from that of the target cell are different from the target cell in the connected shape of the focusing points.

Therefore, it is possible to determine whether or not the cell to be determined for life or death is the target cell on the basis of the connected shape of the focusing points.

### -Feature Amount 9- (Identification of Cell Based on Color Distribution of Focusing Point)

The connected image may include information related to the identification of the cell based on a color distribution of the focusing point.

In a case in which the living cell is irradiated with white light, a blue component (short-wavelength component) is focused at a position closer to the cell than a red component (long-wavelength component). A color distribution is seen at the focusing point of the lens effect. The color distribution of the focusing point of the lens effect reflects the average refractive index of the cell. Therefore, other substances (for example, other cells, red blood cells, and oil droplets) having an average refractive index different from that of the target cell are different from the target cell in the color distribution of the focusing point.

Therefore, it is possible to determine whether or not the cell to be determined for life or death is the target cell on the basis of the color distribution of the focusing point.

For example, in the case of the living cell, the color separation of the focusing point of the lens effect is seen, and a color distribution is seen in which the vicinity of the cell is colored blue and a relatively far portion is colored red. On the other hand, for example, in the case of the red blood cell, the color separation of the focusing point is weak due to a shape like a concave lens, and the focusing point tends to appear relatively white.

For example, for the connected image for analysis, an image of a portion in which the focusing points are connected may be set as the feature amount 9, and whether or not the cell to be determined for life or death is the target cell may be determined by the machine learning device on the basis of the feature amount 9 of the connected image for analysis, the feature amount 8 of the known connected image for reference of the target cell, and the result of determining whether or not the cell is the living cell.

### -Feature Amount 10- (Determination of Whether Cell Is Alive or Dead and Identification of Cell Based on Rate of Match Between Connected Images)

As described above, the connected image includes information related to the determination of whether the cell is alive or dead and the identification of the cell. Therefore, it is possible to determine whether the cell is alive or dead and whether or not the cell to be determined for life or death is the target cell, on the basis of the rate of match between the connected image for analysis and the connected image for reference.

Therefore, the connected image for analysis may be set as the feature amount 10, and whether or not the cell to be determined for life or death is the living cell and whether or not the cell is the target cell may be determined by the machine learning device on the basis of the feature amount 10 of the connected image for analysis, the feature amount 10 of the known connected image for reference of the target cell, and the result of determining whether or not the cell is the living cell.

### -Feature Amount 11- (Determination of Whether Cell Is Alive or Dead Based on Transmission Amount of Lens Effect of Cell)

The connected image includes information related to whether the cell is alive or dead based on the transmission amount of the lens effect of the cell.

The "transmission amount of the lens effect" of the cell means the transmission amount of light through the cell in a case in which the cell has the lens effect.

In the connected image, the "transmission amount of the lens effect" of the cell means a weighted sum of the average brightness of "a plurality of image pieces in the imaging direction" starting from the image piece of the in-focus plane of the cell and the average brightness of "a plurality of image pieces in the direction of the light source" starting from the image piece of the in-focus plane of the cell in a case in which the cell has the lens effect. A weighting coefficient is not particularly limited and may be set as appropriate.

In some cases, the dead cell immediately after the cell membrane is broken has the focusing point caused by the lens effect as an intermediate state, similarly to the living cell. In the dead cell, light is scattered on the outer peripheral surface due to the broken cell membrane. Therefore, the transmission amount of the lens effect is less than that in the living cell. Therefore, it is possible to determine whether the cell is alive or dead on the basis of the transmission amount of the lens effect. It is assumed that the output of the light source is managed to be constant.

The average brightness of the image pieces in the imaging direction with respect to the in-focus plane of the cell is, for example, the average brightness of the image piece S₀ of the in-focus plane P₀ to the image piece Sₙ of the focal plane Pₙ illustrated in Fig. 2.

The average brightness of the image pieces in the direction of the light source with respect to the in-focus plane of the cell is, for example, the average brightness of the image piece S₀ of the in-focus plane P₀ to the image piece S₋ₘ of the focal plane P₋ₘ illustrated in Fig. 2 (for example, n = m is desirable).

The weighted sum of the average brightness of "a plurality of image pieces in the imaging direction" starting from the image piece of the in-focus plane of the cell and the average brightness of "a plurality of image pieces in the direction of the light source" starting from the in-focus plane of the cell may be set as the feature amount 11, and whether the cell is alive or dead may be determined on the basis of a predetermined range of the feature amount 11.

In the case of a multi-wavelength light source (for example, a white LED) and a spectral imaging device (for example, an RGB color camera), a connected image may be created for each color, and the average brightness of "the plurality of image pieces in the imaging direction" and the average brightness of "the plurality of image pieces in the direction of the light source" may be combined from different colors to determine whether the cell is alive or dead.

The feature amounts 1 to 11 described above are examples of the feature amounts obtained from the connected images, and the determination of whether the cell is alive or dead and the identification of the cell may be performed on the basis of other feature amounts.

The feature amount 1 and the feature amount 11 are related to the lens effect of the cell. In addition, the feature amounts 2 to 5 are related to the average refractive index of the cell. Further, the feature amount 6 is related to the diameter of the cell. Furthermore, the feature amount 7 is related to the specific gravity of the cell. Moreover, the feature amount 8 and the feature amount 9 are related to the form of the connected image. Further, the feature amount 10 is related to all of the average refractive index of the cell, the diameter of the cell, and the specific gravity of the cell.

As described above, since many feature amounts can be obtained from the connected image, the connected image is effective not only for determining whether the cell is alive or dead but also for identifying the cell.

It is preferable that the feature amounts used for determining whether the target cell is alive or dead include one or more feature amounts selected from the group consisting of the feature amounts 1 to 11, that is, one or more feature amounts selected from the group consisting of the feature amount related to the lens effect of the cell, the feature amount related to the average refractive index of the cell, the feature amount related to the diameter of the cell, and the feature amount related to the specific gravity of the cell.

### -Life-and-Death Determination Step-

In the life-and-death determination step, it is determined whether the plurality of target cells in the cell suspension are alive or dead on the basis of the feature amount of the connected image for analysis and a predetermined range of the feature amount. In another embodiment, it may be determined whether the target cells are alive or dead on the basis of the feature amount of the known connected image for reference of the cell and the result of determining whether or not the target cell is the living cell.

The determination of whether the target cell is alive or dead includes the determination of whether or not the cell to be determined for life or death is the target cell, that is, the identification of the cell.

The "predetermined range of the feature amount" means a threshold value for distinguishing whether or not the cell is the living cell. In a case in which the feature amount is within the above-described range, it is possible to determine that the cell is the living cell. A method for determining the "predetermined range of the feature amount" is not particularly limited and may be appropriately determined such that whether the cell is alive or dead can be determined.

In another embodiment, whether the cell is alive or dead may be determined on the basis of the feature amount of the known connected image for reference of the cell and the result of determining whether or not the cell is the living cell. This aspect is preferable in a case in which whether the cell is alive or dead is determined by the machine learning device.

Whether the target cell is alive or dead may be determined, using one or more of the feature amounts 1 to 3 and the feature amount 11 from the viewpoint of determining whether the target cell is alive or dead and using one or more of the feature amounts 4 to 9 from the viewpoint of identifying the cell.

From the viewpoint of further improving the accuracy of the determination of whether the cell is alive or dead, whether the cell is alive or dead may be determined using a combination of two or more of the feature amounts 1 to 3 and the feature amount 11.

The determination may be performed using any one of the feature amounts 4 to 9. From the viewpoint of further improving the accuracy of the identification of the cell, the identification of the cell may be performed using a combination of two or more of the feature amounts 4 to 9.

In another embodiment, whether the target cell is alive or dead may be determined using the feature amount 10 on the basis of the rate of match between the connected images. The determination of whether or not the cell to be determined for life or death is the target cell is performed at the same time as the determination of whether the cell is alive or dead.

In a certain embodiment, in a case in which whether the cell is alive or dead is determined using the feature amount 2 (or the feature amount 4) based on the start position of the focusing point, the feature amount 2 can also function the feature amount 4 (or the feature amount 2) based on the start position of the focusing point to determine whether or not the cell to be determined for life or death is the target cell.

In another embodiment, in a case in which whether the cell is alive or dead is determined using the feature amount 3 (or the feature amount 4) based on the continuous length of the focusing point, the feature amount 3 can also function as the feature amount 4 (or the feature amount 3) based on the continuous length of the focusing point to determine whether or not the cell to be determined for life or death is the target cell.

### (Machine Learning Device)

Whether the target cell is alive or dead may be determined using the machine learning device. The machine learning device may be constructed by any one of a neural network, a support vector machine, or a boosting method. It is preferable that the machine learning device is constructed by the neural network and subjected to deep learning.

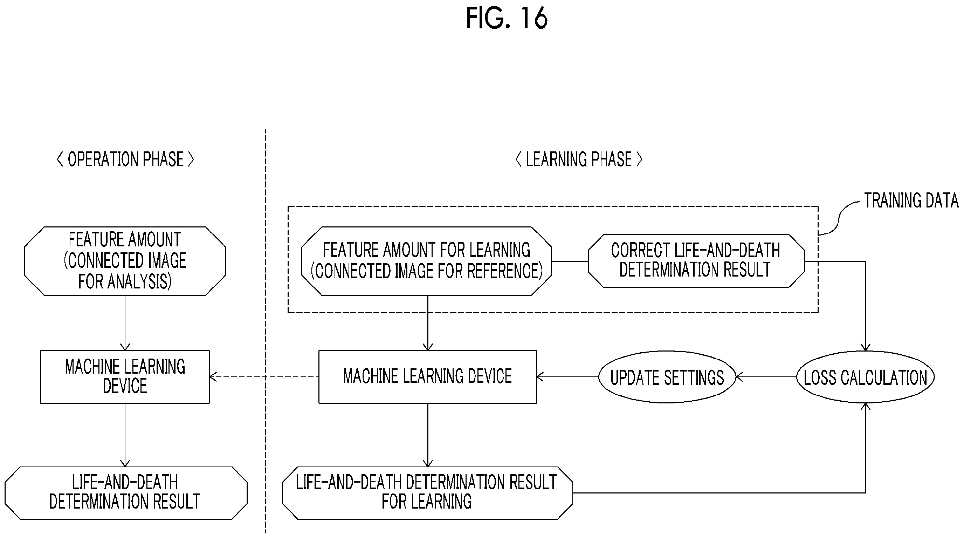

As illustrated in Fig. 16, in a learning phase, the machine learning device is given training data and then trained. The training data is a set of the feature amount of the known connected image for reference of the target cell (hereinafter, sometimes referred to as a "feature amount for learning") and a result of determining whether or not the cell is the living cell corresponding to the feature amount for learning (hereinafter, sometimes referred to as a "correct life-and-death determination result").

In the learning phase, the feature amount for learning is input to the machine learning device. The machine learning device outputs a life-and-death determination result for learning with respect to the feature amount for learning. The loss calculation of the machine learning device using a loss function is performed on the basis of the life-and-death determination result for learning and the correct life-and-death determination result. Then, update settings of various coefficients of the machine learning device are made according to the result of the loss calculation, and the machine learning device is updated according to the update settings.

In the learning phase of the machine learning device, the series of processes of the input of the feature amount for learning to the machine learning device, the output of the life-and-death determination result for learning from the machine learning device, the loss calculation, the update setting, and the update of the machine learning device is repeated performed while the training data is being exchanged. The repetition of the series of processes is ended in a case in which the prediction accuracy of the life-and-death determination result for learning with respect to the correct life-and-death determination result has reached a predetermined set level. The machine learning device whose prediction accuracy has reached the set level is used in an operation phase and outputs the life-and-death determination result in response to the input of the feature amount of the connected image for analysis.

In the life-and-death determination step, in order to determine whether the plurality of cells in the cell suspension are alive or dead, a connected image for analysis is created for the plurality of cells in the cell suspension. Then, whether the target cell is alive or dead may be determined on the basis of the feature amounts of these connected images for analysis, the feature amount of the known connected image for reference of the target cell, and the result of determining whether or not the target cell is the living cell.

In addition, for example, the connected images for analysis of the target cell and other substances (for example, other cells, red blood cells, and oil droplets) are created using the cell suspension including the target cell and other substances. Then, the identification of the target cell and the determination of whether the target cell is alive or dead may be performed at the same time on the basis of the feature amounts of these connected images for analysis, the feature amount of the known connected image for reference of the target cell, and the result of determining whether or not the target cell is the living cell.

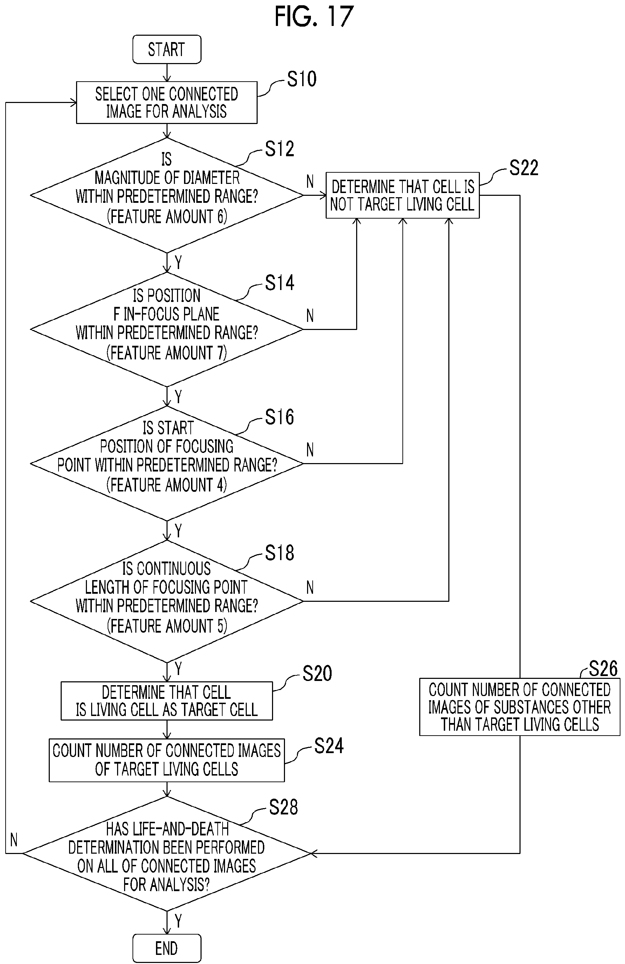

An example of a life-and-death determination flow of the life-and-death determination step will be described with reference to a flowchart illustrated in Fig. 17. The life-and-death determination flow illustrated in Fig. 17 is an example in which the target cell is identified using the feature amounts 4 to 7 and whether the target cell is alive or dead is determined using the feature amounts 4 and 5.

The life-and-death determination flow illustrated in Fig. 17 is an example in which the target cell is identified using the feature amounts 4 to 6 and whether the target cell is alive or dead is determined using the feature amounts 4 and 5.

### -Step S10-

One connected image for analysis is selected from a plurality of connected images for analysis (S10).

### -Step S12-

It is determined whether or not the feature amount 6 (the magnitude of the diameter) of the connected image for analysis is within a predetermined range of the feature amount 6 (S12). In a case in which the feature amount 6 of the connected image for analysis is within the above-described range, it is determined that the cell is the target cell, and the process proceeds to the next step (S14).

In a case in which the feature amount 6 of the connected image for analysis is not within the above-described range, it is determined that the cell is not the target cell (S22).

### -Step S14-

It is determined whether or not the feature amount 7 (the position of the in-focus plane) of the connected image for analysis is within a predetermined range of the feature amount 7 (S14). In a case in which the feature amount 7 of the connected image for analysis is within the above-described range, it is determined that the cell is the target cell, and the process proceeds to the next step (S16).

In a case in which the feature amount 7 of the connected image for analysis is not within the above-described range, it is determined that the cell is not the target cell (S22).

### -Step S16-

It is determined whether or not the feature amount 4 (the start position of the focusing point) of the connected image for analysis is within a predetermined range of the feature amount 4 (S16). In a case in which the feature amount 4 of the connected image for analysis is within the above-described range, it is determined that the cell is a living cell as the target cell (hereinafter, sometimes referred to as a "target living cell"), and the process proceeds to the next step (S18).

In a case in which the feature amount 4 of the connected image for analysis is not within the above-described range, it is determined that the cell is not the living cell as the target cell (S22).

### -Step S18-

It is determined whether or not the feature amount 5 (the continuous length of the focusing point) of the connected image for analysis is within a predetermined range of the feature amount 5 (S18). In a case in which the feature amount 5 of the connected image for analysis is within the above-described range, it is determined that the cell is the living cell as the target cell (S20).

In a case in which the feature amount 5 of the connected image for analysis is not within the above-described range, it is determined that the cell is not the living cell as the target cell (S22).

### -Step S20 and Step S22-

In a case in which it is determined that the cell is the living cell as the target cell (S20), the process proceeds to the next step (S24). In a case in which it is determined that the cell is not the living cell as the target cell (S22), the process proceeds to the next step (S26).

### -Step S24-

The number of connected images in which the cell has been determined to be the living cell as the target cell is counted, and the process proceeds to the next step (S28).

### -Step S26-

The number of connected images in which the cell has been determined not to be the living cell as the target cell is counted, and the process proceeds to the next step (S28).

### -Step S28-

In a case in which there is a connected image for analysis in which whether the target cell is alive or dead has not been determined, the process returns to Step S10. In a case in which whether the target cell is alive or dead has been determined for all of the connected images for analysis, the life-and-death determination flow is ended.

### -Living Cell Concentration Determination Step-

In the living cell concentration determination step, the living cell concentration of the target cells in the cell suspension is determined on the basis of the result of determining whether the target cells are alive or dead.

In the present disclosure, the living cell concentration of the target cells means the number of living cells per unit volume [cells/ml].

For example, the living cell concentration of the target cells can be calculated as follows.

The cell suspension is accommodated in the holding container, a connected image for analysis is created for all of the target cells (which may include substances (for example, other cells, red blood cells, and oil droplets) other than the target cells) present between the bottom of the holding container and the surface of the cell suspension in a randomly selected field of view, it is determined whether the target cells are alive or dead, and the number of connected images for analysis in which the target cell has been determined to be the living cell is used as the number of target living cells. The number of living cells as the target cells can be divided by the volume of the cell suspension used for measurement (that is, the product of the area of the field of view and the height of the cell suspension (the height from the bottom surface of the holding container to the liquid surface)) to calculate the living cell concentration of the target cells in the cell suspension.

### (Living Cell Concentration Measurement Step According to Aspect 2)

The living cell concentration measurement step according to Aspect 2 includes: a step of imaging the target cell with a digital holographic microscope to acquire a phase image of the target cell (hereinafter, sometimes referred to as a "phase image acquisition step"); a step of determining whether the plurality of target cells in the cell suspension are alive or dead on the basis of a phase amount distribution of the phase image and a predetermined phase amount distribution (hereinafter, sometimes referred to as a "life-and-death determination step"); and a step of determining the living cell concentration of the target cells in the cell suspension on the basis of a result of determining whether the target cells are alive or dead (hereinafter, sometimes referred to as a "living cell concentration determination step").

### -Phase Image Acquisition Step-

In the phase image acquisition step, the target cell is imaged by the digital holographic microscope to acquire a phase image of the target cell.

The phase image of the target cell can be acquired by an interference pattern generated by light passing through the target cell and reference light.

The digital holographic microscope is not particularly limited, and a known digital holographic microscope may be used.

### -Life-and-Death Determination Step-

In the phase image acquisition, whether the plurality of target cells in the cell suspension are alive or dead is determined on the basis of the phase amount distribution of the phase image of the target cell and a predetermined phase amount distribution.

The living cell has a refractive index distribution inside the cell, but the dead cell has a smaller refractive index distribution than the living cell. Therefore, the phase image of the target cell obtained by the digital holographic microscope has a phase amount distribution unique to the living cell.

The "phase amount distribution" has information related to the refractive index distribution inside the cell and information related to the size of the cell.

The "predetermined range of the phase amount distribution" means a threshold value for distinguishing whether or not the cell is the living cell as the target cell. In a case in which the phase amount distribution is within the above-described range, it is possible to determine that the cell is the living cell as the target cell. A method for determining the "predetermined range of the phase amount distribution" is not particularly limited and may be appropriately determined such that whether the target cell is alive or dead can be determined.

In addition, for example, whether the target cell is alive or dead may be determined on the basis of the phase amount distribution of the captured phase image, a known phase amount distribution of the target cell, and the result of determining whether or not the target cell is the living cell.

### -Living Cell Concentration Determination Step-

In the living cell concentration determination step, the living cell concentration of the target cells in the cell suspension is determined on the basis of the result of determining whether the target cells are alive or dead. The details are the same as those described in the living cell concentration measurement step according to Aspect 1.

### [Liquid Amount Determination Step]

In the liquid amount determination step, the amount of cell suspension to be seeded and the amount of diluent required to adjust the amount of cell suspension to be seeded to a predetermined living cell concentration are determined on the basis of the living cell concentration and the amount data of the cell suspension.

The amount of cell suspension and the amount of diluent may be appropriately determined such that the living cell concentration of the diluted cell suspension has a desired value.

### [Diluting Step]

In the diluting step, the determined amount of cell suspension and the determined amount of diluent are mixed. A mixing unit is not particularly limited and may be appropriately selected.

In this way, the living cell concentration of the target cells can be rapidly adjusted by the living cell concentration adjustment method according to the present disclosure.

### [Cell Survival Rate Determination Step]

The living cell concentration adjustment method may include a step of determining a cell survival rate of the plurality of target cells in the cell suspension on the basis of a result of determining whether the plurality of target cells in the cell suspension are alive or dead (hereinafter, sometimes referred to as a "cell survival rate determination step").

The cell survival rate is a value obtained by dividing the number of living cells by the total number of cells (the sum of the number of living cells and the number of dead cells).

In a case in which the living cell concentration measurement step according to Aspect 1 is performed, the cell survival rate can be calculated by setting the number of connected images for analysis in which the target cell has been determined to be the living cell (for example, Step S24 in Fig. 17) as the number of living cells and by setting the total number of connected images for analysis (for example, Steps S24 and S26 in Fig. 17) as the total number of cells.

In a case in which the living cell concentration measurement step according to Aspect 2 is performed, the cell survival rate can be calculated from the number of cells determined to be the living cells and the total number of cells determined to be alive or dead (the total number of cells).

In subculture and expansion culture, the cell suspension includes only one type of target cell. Therefore, the cell survival rate obtained in this case is the ratio of the target living cells to all of the target cells.

The disclosure of JP2021-076027 filed on April 28, 2021 is incorporated herein by reference in its entirety. All documents, patent applications, and technical standards disclosed in this specification are incorporated in this specification by reference such that the incorporation of each of the documents, the patent applications, and the technical standards by reference is specific and is as detailed as each of the documents, the patent applications, and the technical standards.

## Claims

1. A cell culture apparatus comprising:
a first container that holds a cell suspension;
a second container that holds a diluent diluting the cell suspension;
a living cell concentration measurement device that individually determines whether a plurality of target cells in the cell suspension are alive or dead to measure a living cell concentration of the target cells in the cell suspension;
a control device that holds and controls the living cell concentration and amount data of the cell suspension;
a mixing unit that mixes the cell suspension and the diluent; and
a culture container in which the diluted cell suspension is seeded to culture cells,
wherein the control device determines an amount of the cell suspension to be seeded and an amount of the diluent required to adjust the cell suspension to be seeded to a predetermined living cell concentration, on the basis of the living cell concentration and the amount data of the cell suspension, feeds the amount of the cell suspension and the amount of the diluent to the mixing unit, and seeds the diluted cell suspension.

2. The cell culture apparatus according to claim 1,
wherein the living cell concentration measurement device is disposed in a flow path that is branched from a flow path between the first container and the mixing unit or in the flow path between the first container and the mixing unit.

3. The cell culture apparatus according to claim 2,
wherein the living cell concentration is measured for at least a portion of a liquid feeding time until the cell suspension reaches the mixing unit from the first container.

4. The cell culture apparatus according to any one of claims 1 to 3,
wherein the mixing unit also serves as the culture container.

5. The cell culture apparatus according to any one of claims 1 to 4,
wherein the mixing unit also serves as the culture container which is a suspension culture apparatus.

6. The cell culture apparatus according to any one of claims 1 to 3,
wherein the mixing unit also serves as the second container.

7. The cell culture apparatus according to any one of claims 1 to 6,
wherein the culture container is a multi-layer culture container.

8. The cell culture apparatus according to any one of claims 1 to 7,
wherein the living cell concentration measurement device includes:
a light source that irradiates the target cell with light;
an imaging device that images the target cell;
a unit that changes a focal plane;
an image acquisition unit that acquires images of the target cell captured in a plurality of focal planes including an in-focus plane of the target cell in a direction opposite to a side on which the target cell is irradiated with the light;
an image piece acquisition unit that acquires an image piece including a central portion and an outer peripheral portion of the target cell from each of the images;
a connected-image-for-analysis creation unit that connects the image pieces in an order of an imaging direction of the focal plane to create a connected image for analysis;
a feature amount extraction unit that extracts a feature amount from the connected image for analysis;
a life-and-death determination unit that determines whether the plurality of target cells in the cell suspension are alive or dead on the basis of the feature amount of the connected image for analysis and a predetermined range of the feature amount; and
a living cell concentration determination unit that determines the living cell concentration of the target cells in the cell suspension on the basis of a result of determining whether the target cells are alive or dead.

9. The cell culture apparatus according to claim 8,
wherein the unit changing the focal plane is a stage moving mechanism that moves a stage, on which a holding container holding the target cell is placed, to change a distance between the target cell and the imaging device.

10. The cell culture apparatus according to claim 8,
wherein the unit changing the focal plane is an imaging device moving mechanism that moves the imaging device to change a distance between the target cell and the imaging device.

11. The cell culture apparatus according to claim 8,
wherein the imaging device includes a liquid lens as the unit changing the focal plane.

12. The cell culture apparatus according to any one of claims 1 to 7,
wherein the living cell concentration measurement device includes:
a digital holographic microscope that images the target cell;
a phase image acquisition unit that acquires a phase image of the target cell;
a life-and-death determination unit that determines whether the plurality of target cells in the cell suspension are alive or dead on the basis of a phase amount distribution of the phase image and a predetermined phase amount distribution; and
a living cell concentration determination unit that determines the living cell concentration of the target cells in the cell suspension on the basis of a result of determining whether the target cells are alive or dead.

13. The cell culture apparatus according to any one of claims 1 to 12,
wherein the living cell concentration measurement device includes a cell survival rate determination unit that determines a cell survival rate of the target cells in the cell suspension on the basis of a result of determining whether the target cells are alive or dead.

14. A living cell concentration adjustment method comprising:
individually determining whether a plurality of target cells in a cell suspension are alive or dead to measure a living cell concentration of the target cells in the cell suspension;
determining an amount of the cell suspension to be seeded and an amount of a diluent required to adjust the cell suspension to be seeded to a predetermined living cell concentration, on the basis of the living cell concentration and amount data of the cell suspension; and
mixing the amount of the cell suspension and the amount of the diluent.

15. The living cell concentration adjustment method according to claim 14,
wherein the measuring of the living cell concentration includes:
acquiring images of the target cell captured in a plurality of focal planes including an in-focus plane of the target cell in a direction opposite to a side on which the target cell is irradiated with the light;
acquiring an image piece including a central portion and an outer peripheral portion of the target cell from each of the images;
connecting the image pieces in an order of an imaging direction of the focal plane to create a connected image for analysis;
extracting a feature amount from the connected image for analysis;
determining whether the plurality of target cells in the cell suspension are alive or dead on the basis of the feature amount of the connected image for analysis and a predetermined range of the feature amount; and
determining the living cell concentration of the target cells in the cell suspension on the basis of a result of determining whether the target cells are alive or dead.

16. The living cell concentration adjustment method according to claim 14,
wherein the measuring of the living cell concentration includes:
imaging the target cell with a digital holographic microscope to acquire a phase image of the target cell;
determining whether the plurality of target cells in the cell suspension are alive or dead on the basis of a phase amount distribution of the phase image and a predetermined phase amount distribution; and
determining the living cell concentration of the target cells in the cell suspension on the basis of a result of determining whether the target cells are alive or dead.

17. The living cell concentration adjustment method according to any one of claims 14 to 16, further comprising:
determining a cell survival rate of the target cells in the cell suspension on the basis of a result of determining whether the target cells are alive or dead.
